# EUROPEAN PATENT APPLICATION

(11) **EP 2 402 362 A2**
(43) Date of publication of application: **04.01.2012**
(21) Application number: 11155898.7
(22) Date of filing: 28.04.2005
(51) Int. Cl.: C07J 71/00, A61K 31/58, A61P 19/10, A61P 25/28

(54) **Crystalline forms of smilagenin**

(30) Priority: 28.04.2004 GB 0409567
(62) Divisional of application: 05738225.1
(71) Applicant: Phytopharm PLC, Ermine Business Park Huntingdon Cambridgeshire PE29 6UA (GB)
(72) Inventor: Tiffin, Peter David, Huntingdon, Cambridgeshire PE29 6UA (GB)
(74) Representative: Barnes, Colin Lloyd

(57) **Abstract**

The invention provides smilagenin in novel crystalline, hydrated forms, and the use thereof in manufacturing pharmaceutical or edible grade smilagenin and its derivatives.

## Description

### Field of the Invention

The present invention relates to novel amorphous and crystalline forms of smilagenin and its hydrates.

### Background to the Invention

It is well established that some organic compounds can crystallise in a number of different polymorphic forms or crystal habits, which may comprise the compound as such, solvates of the compound, hydrates of the compound, or combinations thereof. Alternatively, the compound, solvate or hydrate may precipitate as an amorphous solid.

The stability and bioavailability of the drug product may vary according to the polymorphic form present. The choice of crystal form is thus a critical aspect of drug development (Brittain, Pharm. Tech. pp. 50-52, 1994; Yu et al., Pharm. Sci. Techno/. Today, 1, pp. 118 to 127, 1998; Byrn et al., Chem. Mater. 6, pp. 1148 to 1158, 1994; Byrn et al., Pharm. Res. , 12, pp. 945 to 954, 1995; Henk et al., Pharm. Ind. 59, pp. 165 to 169, 1997).

Smilagenin is an A/B-*cis* steroidal sapogenin having the formula:

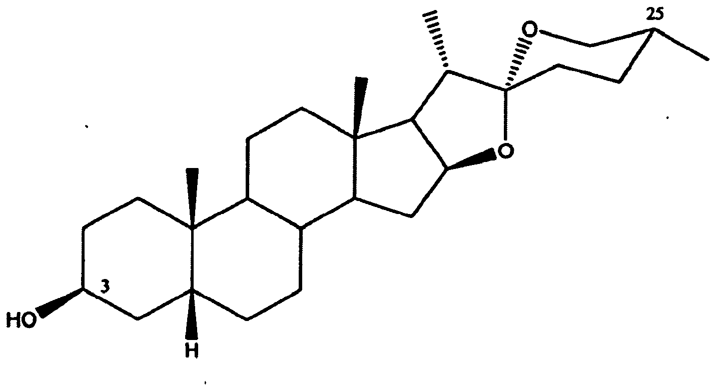

Smilagenin and its derivatives have been identified as valuable therapeutic agents in human and veterinary medicine and in non-therapeutic human and non-human animal treatments. See, for example, US Patent No. 3890438 (use of smilagenin and certain 4-substituted phenoxyisobutyric acid compounds against high blood cholesterol levels); US Patent No. 4680289 (use of smilagenin against obesity and diabetes obesity syndromes); US Patent No. 6258386 (use of smilagenin against cognitive dysfunction and allied conditions); WO-A-01/23406, WO-A-01/23407, WO-A-01/23408, and WO-A-O1/49703 (use of smilagenin derivatives against cognitive dysfunction and allied conditions); and WO-A-02/079221 and WO-A-03/082893 (use of smilagenin and derivatives thereof against non-cognitive neurodegeneration, non-cognitive neuromuscular degeneration, motor-sensory neurodegeneration and loss of receptor function in the absence of cognitive, neural or neuromuscular impairment).

In a key article (Marker et al., J. Am. Chem. Soc. 65, pp. 1199 to 1209, 1943, at p. 1207), it was reported that smilagenin acetate shows polymorphic forms melting at 110, 130 and 152 °C. The melting point of smilagenin from a number of sources was always in the range 183-185°C. However, the recrystallisation solvent was not stated and the article made no mention of polymorphic forms of smilagenin.

*In* J. Am. Chem. Soc. pp. 2525 to 2532, 1940, Marker *et al.* reported a melting point of 183-185°C for smilagenin crystallised from alcohol.

In J. Am. Chem. Soc. 64, pp. 818 to 822, 1942, Marker *et al.* reported a melting point of 178-180°C for smilagenin crystallised from acetone.

Askew *et al.* reported that fractional crystallisation of smilagenin from acetone gave long silky needles with a melting point of 183-184°C. It was further reported that smilagenin appeared to form a hydrate when crystallised from methanol (Askew et al., J. Chem. Soc. pp. 1399 to 1403, 1936). However, no evidence was provided to support this observation, and the reader was merely referred back to an earlier paper on a related compound (Power et al., J. Chem. Soc., 105, pp. 201 to 219, 1914).

Scheer *et al.* crystallised smilagenin from aqueous ethanol and observed a melting point of 187-188°C, but made no mention of the formation of a hydrate (Scheer et al., J. Am. Chem. Soc., 77, pp. 641 to 646, 1955).

*In* J. Am. Chem. Soc., 77, pp. 3086 to 3089, 1955, Wall *et al.* commented that the best samples of smilagenin from natural sources had a melting point of 188-189°C, whereas acetone or aqueous acetone failed to bring the melting point above 182-185°C on samples generated by isomerization of sarsasapogenin. Again, no mention was made of hydrate formation. The infra-red (IR) spectra of the synthetic and natural materials were identical. However, the X-ray powder diffraction (XRPD) patterns were not. These differences were not attributed to polymorphism, and the XRPD patterns were not presented. The authors concluded that there was a pronounced effect of traces of sarsasapogenin on certain properties of smilagenin that depend on crystal structure.

Wall et al., (J. Biol. Chem., 198, pp. 533 to 543, 1952) reported the melting point of smilagenin to be 184°C. However, the recrystallisation solvent was not stated. The paper reported that the use of a Kofler microscopic melting point apparatus having polarizing disks allowed for the crystal form or habit to be observed. No mention was made of polymorphism but the impact of impurities upon the melting point was noted. In J. Am. Chem. Soc., 77, pp. 1230 to 1237, 1954, Wall *et al.* reported the melting point of smilagenin to be 183°C.

Callow et al. (J. Am. Chem. Soc. 77, p. 1672, 1955) described the recrystallisation of smilagenin from acetone to yield crystals having melting point 157-160 °C.

Parsons et al. (Henry Ford Hosp. Med. Bull., 12, pp. 87 to 120, 1964) described a specific crystalline form of smilagenin by XRPD. From the data presented it cannot be concluded that this form corresponds to any of the forms described herein.

In US Patent No. 3169959 (1965), a melting point of 178-180°C was reported for smilagenin crystallised from heptane.

In Phytochemistry, 8, pp. 1523 to 1531, 1969, Blunden *et al.* reported a melting point of 181-182°C for smilagenin crystallised from acetone.

In J. Nat. Prod., 44, pp. 441 to 447, 1981, Blunden *et al.* reported a melting point of 186°C for smilagenin crystallised from acetone.

The use of crystalline intermediate complexes to assist the extraction of relatively pure smilagenin and other sapogenins from their plant sources has been proposed. Thus, for example, US Patent No. 5017562 described a crystalline saponin-containing complex, derived from the saponin-containing plants *Agave, Yucca, Dioscorea, Quillaja, Medicago* and *Cyamopsis,* which is substantially free of fats and non-saponin carbohydrates and which, on hydrolysis, can yield smilagenin and other sapogenins.

The prior art publications acknowledged above are incorporated herein by reference.

Depending on the administration route desired in the therapy, it may be desirable to improve or at least control the stability and water solubility of the smilagenin, to obtain a desired bioavailability profile. Furthermore, it can assist the manufacturing or purification process if the stability and water solubility of the smilagenin can be controlled.

In principle, the water solubility of polymorphic forms of an organic compound is not necessarily the same for all forms. Therefore, the use of specific crystalline forms or habits can offer useful control of the water solubility. In the case of sparingly watersoluble compounds such as smilagenin, even a slight adjustment to the water-solubility by means of an adjustment to the polymorphic form can offer useful processing or biological advantages.

We have examined commercially available smilagenin and have found that it occurs in a specific crystalline form, which we have characterised as form II.

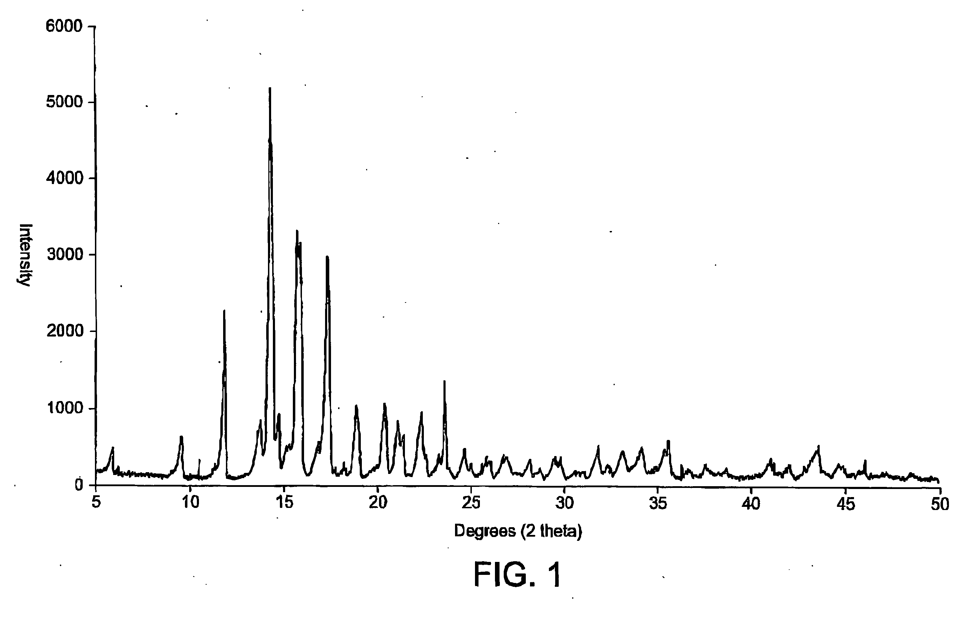

Figure 1 of the accompanying drawings shows an XRPD pattern obtained at λ = 1.5406 Angstroms from a sample of commercially available smilagenin obtained from Research Plus, Inc. This is an example of form II crystalline smilagenin. The intensities of the XRPD pattern for this crystalline form in the 2-theta range 5 to 50 degrees are shown in Table A below, in the column headed "Form II". These data are the intensities at regularly spaced 2-theta values, and are to be used in conjunction with the Figure of the drawings. From these data, the d-spacings may readily be calculated using the Bragg equation. For present purposes, the approximately 20 strongest peaks may generally be considered characteristic of the crystalline form, subject however to standard practice in crystallography.

The present invention is based on our surprising finding that at least three further crystalline forms of smilagenin exist, two of which appear typically to be anhydrous and have been characterised as form I and form III. We have further found that form III can exist in at least one variant form in which the crystal structure is modified ("form IIIA") and/or the hydration level is modified (smilagenin hemihydrate or smilagenin dihydrate). Furthermore, we have surprisingly found that smilagenin can form a crystalline monohydrate. We have characterised the typical crystalline form of this monohydrate as form V. Furthermore, we have surprisingly found that smilagenin can form a crystalline channel hydrate having variable smilagenin:water stoichiometry. We have characterised the typical crystalline form of this channel hydrate as form VL It has also been found that smilagenin can be obtained in a non-crystalline form (the "amorphous form"). The prior art does not describe any amorphous form of smilagenin.

Furthermore, we have identified a crystalline solvate of smilagenin formed with *iso-*propyl alcohol. We have characterised the typical crystalline form of this iso-propyl alcohol solvate as form VII.

Surprisingly, we have found that form II of smilagenin can be converted to the monohydrate by a solvent mediated transformation in hexane or heptane. Further, we have found that the monohydrate may be obtained by other processes, including solvent mediated transformations in aqueous acetone, aqueous tetrahydrofuran and aqueous ethanol. The water content was determined by Karl Fischer analysis was found to be in the range about 3 to 6% w/w. Analysis by thermogravimetric analysis (TGA) confirms a water weight in the region of 4%.

Furthermore, we have found methods of controlling the transitions between not only the novel amorphous and crystalline forms but between them and the known form II, by controlling the organic solvent used for recrystallisation.

Surprisingly, and advantageously, we have found that precipitation or crystallisation of smilagenin from certain organic solvents does not result in organic solvates of smilagenin. However, the iso-propyl alcohol solvate (believed to be a hemi-solvate) can be obtained under certain circumstances, and is a newly recognised material.

These novel forms of smilagenin and associated methods therefore offer enhanced control of the preparation of pharmaceutical or edible grade smilagenin, and the possibility of preparing pharmaceutical or edible grade smilagenin with improved delivery and bioavailability characteristics.

### Brief Description of the Invention

According to a first aspect of the present invention, there is provided smilagenin in any one or more of crystalline forms I, III, IIIA, V, VI and VII as defined herein.

According to an example of this first aspect of the present invention, there is provided smilagenin in any one or more of crystalline forms I, III, IIIA and V as defined herein.

According to a second aspect of the present invention, there is provided smilagenin hemihydrate, optionally in crystalline form.

According to a third aspect of the present invention, there is provided smilagenin monohydrate, optionally in crystalline form.

According to a fourth aspect of the present invention, there is provided smilagenin dihydrate, optionally in crystalline form.

According to a fifth aspect of the present invention, there is provided smilagenin channel hydrate, optionally in crystalline form.

According to a sixth aspect of the present invention, there is provided amorphous smilagenin.

According to a seventh aspect of the present invention, there is provided smilagenin iso-propyl alcohol solvate, optionally in crystalline form. This material may be prepared by precipitation from a solution of relatively impure smilagenin in iso-propyl alcohol that has been reduced in volume by azeotropic distillation.

According to an eighth aspect of the present invention, there is provided crystalline pharmaceutical or edible grade anhydrous unsolvated smilagenin, when obtained by crystallisation from a solution of substantially pure smilagenin iso-propyl alcohol (IPA) solvate in an anhydrous IPA-compatible organic solvent which does not form a solvate with smilagenin, such as acetone. The smilagenin may be in crystalline form I or III as defined herein.

The crystalline or amorphous material of the present invention may be present substantially free of other forms of smilagenin and/or substantially free of other steroidal sapogenins and/or steroidal saponins.

The crystalline or amorphous material of the present invention may preferably be present in at least about 50% by weight pure form, for example at least about 70% by weight pure form, for example at least about 80% by weight pure form, for example at least about 85% by weight pure form, for example at least about 90% by weight pure form, for example at least about 95% by weight pure form, for example at least about 97% by weight pure form, for example at least about 98% by weight pure form.

Any of the materials according to the present invention may if desired be present in admixture with one or more other materials according to the present invention, another form of smilagenin, any other biologically active material, any biologically inactive material, or any combination thereof. The said other form of smilagenin, when present, may be crystalline form II.

The novel forms of smilagenin provided by the present invention possess a number of advantages over the known form, particularly in terms of their stability and handling characteristics. These advantages are applicable to one or more of the manufacturing, purification, formulation and storage phases of the marketed smilagenin compositions and/or to the delivery of the smilagenin from the composition to the human or non-human animal patient for achieving the desired pharmacological effect.

The present invention also provides methods for preparing the materials of the present invention, preferably by precipitation of smilagenin from a solution of smilagenin in an appropriate organic solvent or solvent mixture or by other crystallisation of smilagenin in an appropriate organic solvent or solvent mixture, optionally in the presence of water, as well as medicaments, foodstuffs and beverages containing the said materials, methods of preparing the medicaments, foodstuffs and beverages, uses of the said materials in the preparation of the medicaments, foodstuffs and beverages, and uses of the medicaments, foodstuffs and beverages in human and veterinary medicine and in non-therapeutic human and non-human animal treatments.

The present invention further provides a process for obtaining pharmaceutical or edible grade smilagenin or a derivative thereof, wherein at least one step of the process includes preparing smilagenin in one or more of the forms according to the present invention. The smilagenin may be prepared in any suitable level of hydration and in any suitable physical form, for example as an isolated dry solid or in a liquid medium such as a crystal slurry.

The resultant pharmaceutical or edible grade smilagenin or derivative thereof may be subsequently formulated into a suitable medicament, foodstuff or beverage form.

The present invention further provides methods of adjusting the crystalline form of smilagenin between the forms I, II, III, IIIA, V, VI and VII (for example between the forms I, II, III, IIIA and V), methods of adjusting the form of smilagenin between its amorphous and crystalline forms, methods of adjusting the hydration level of smilagenin, methods of forming the iso-propyl alcohol solvate of smilagenin and methods of adjusting the form of smilagenin between two or more of the hydrated, solvated and unhydrated and unsolvated forms.

The terms "crystallise", "recrystallise" and the like, used herein, refer to all methods suitable for forming a desired crystal form or habit or mixture or other combination thereof, and are not limiting. For example, crystal slurrying, crystal precipitation, and other solvent mediated crystal transformation, with or without seeding and/or nucleation, are all encompassed by the terms "crystallise", "recrystallise" and the like as used herein.

The materials according to the present invention may therefore conveniently be present in substantially pure isolated form. The materials may suitably be prepared on a kilogram scale.

### Detailed Description of the Invention

### Crystalline Form I

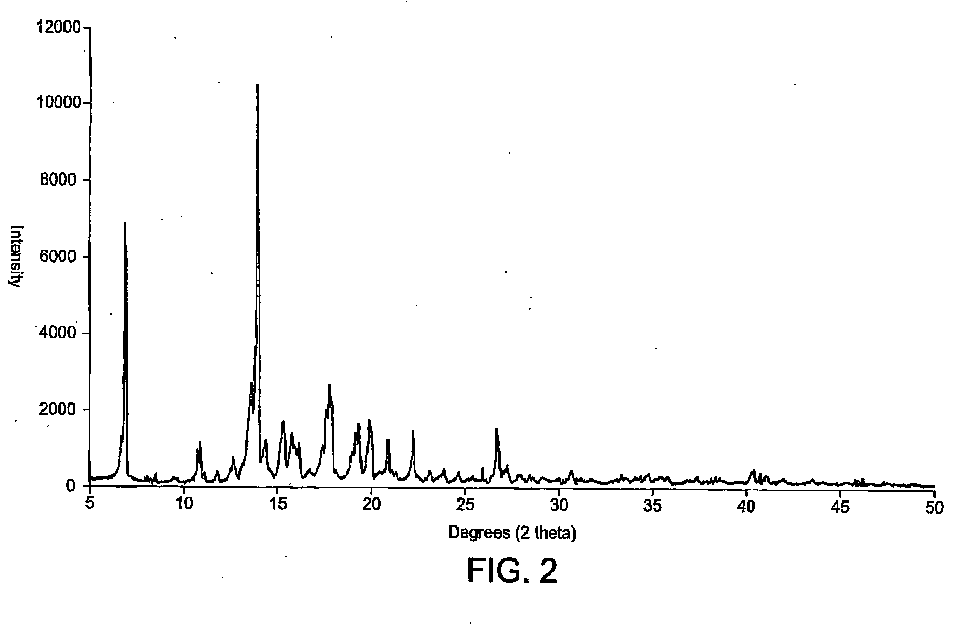

The term "crystalline form I" used herein means that crystalline form of smilagenin which has an XRPD pattern substantially as shown in Figure 2 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees may generally be considered characteristic or diagnostic of the crystalline form, subject however to standard practice in crystallography.

The intensities of the XRPD peaks for this crystalline form in the 2-theta range 5 to 50 degrees are shown in Table A below, in the column headed "Form I". These data are the intensities at regularly spaced 2-theta values, and are to be used in conjunction with the Figure of the drawings. From these data, the d-spacings may readily be calculated using the Bragg equation.

The form I material can be prepared by recrystallisation of commercially available smilagenin from acetone. Alternatively, the form I material can be prepared by a solvent mediated transformation of form II or form III material in acetone, or more preferably in acetonitrile.

Karl Fischer analysis, differential scanning calorimetry and thermogravimetric analysis confirmed that the crystal form I that we have obtained is neither hydrated nor solvated.

### Crystalline Form II

The term "crystalline form II" used herein means that crystalline form of smilagenin which has an XRPD pattern substantially as shown in Figure 1 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees may generally be considered characteristic or diagnostic of the crystalline form, subject however to standard practice in crystallography.

The intensities of the XRPD peaks for this crystalline form in the 2-theta range 5 to 50 degrees are shown in Table A below, in the column headed "Form II". These data are the intensities at regularly spaced 2-theta values, and are to be used in conjunction with the Figure of the drawings. From these data, the d-spacings may readily be calculated using the Bragg equation.

The form II material was commercially available smilagenin obtained from Research Plus, Inc.

Differential scanning calorimetry (DSC), TGA, residual solvent and Karl Fischer analysis confirmed that the material was neither hydrated nor solvated.

### Crystalline Form III

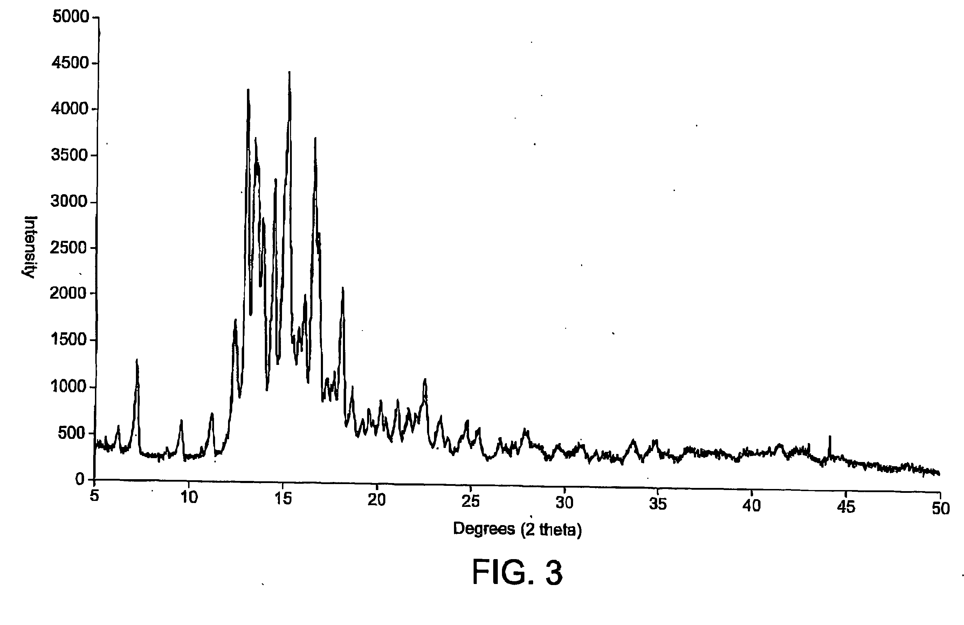

The term "crystalline form III" used herein means that crystalline form of smilagenin which has an XRPD pattern substantially as shown in Figure 3 of the accompanying drawings (λ =1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees may generally be considered characteristic or diagnostic of the crystalline form, subject however to standard practice in crystallography.

The intensities of the XRPD peaks for this crystalline form in the 2-theta range 5 to 50 degrees are shown in Table A below, in the column headed "Form III". These data are the intensities at regularly spaced 2-theta values, and are to be used in conjunction with the Figure of the drawings. From these data, the d-spacings may readily be calculated using the Bragg equation.

The form III material may be prepared by solvent mediated transformation of commercially available smilagenin using methyl t-butyl ether, acetone, methyl *iso*-butyl ketone, ethyl acetate, *iso*-propyl acetate and toluene.

Solvent mediated transformations of commercially available smilagenin using butanone or from 50% aqueous ethanol yielded a mixture of crystal forms III and V.

These data show that crystal form II can be converted into form III by solvent mediated transformations using a range of solvents.

Karl Fischer analysis, differential scanning calorimetry and thermogravimetric analysis confirmed that the crystal form III that we have obtained is neither hydrated nor solvated.

### Crystalline Form IIIA

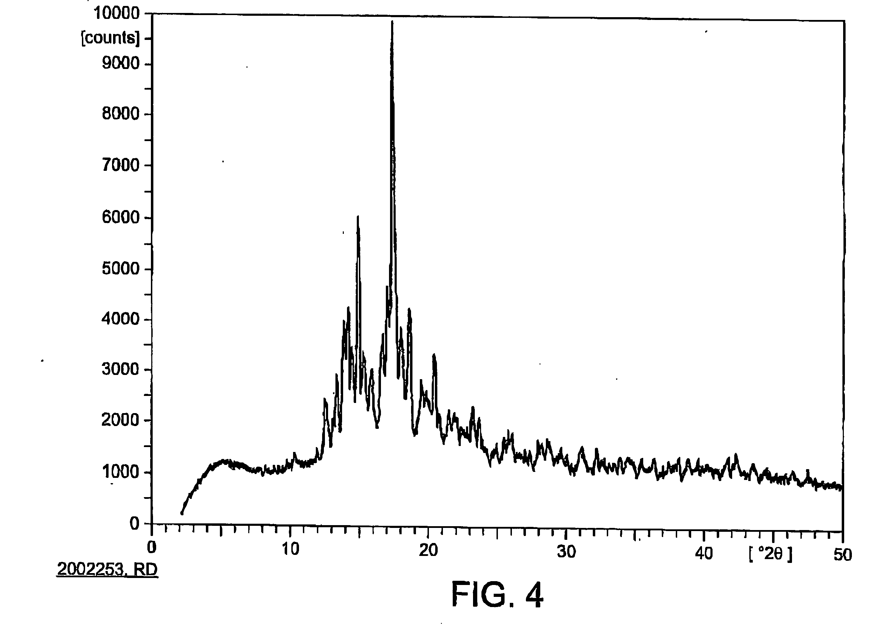

The term "crystalline form IIIA" used herein means that crystalline form of smilagenin which has an XRPD pattern substantially as shown in Figure 4 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees may generally be considered characteristic or diagnostic of the crystalline form, subject however to standard practice in crystallography.

The d-spacings may readily be calculated from the information in Figure 4, using the Bragg equation.

The form IIIA material may be prepared by a solvent mediated transformation of commercially available smilagenin using dimethylformamide.

### Crystalline Form V

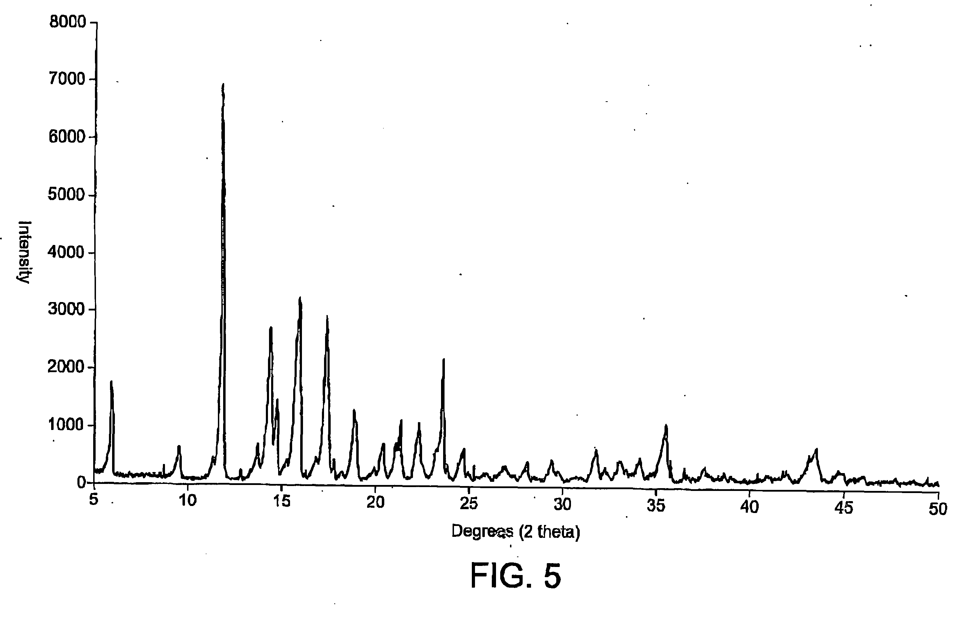

The term "crystalline form V" used herein means that crystalline form of smilagenin which has an XRPD pattern substantially as shown in Figure 5 of the accompanying drawings (λ =1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 5 to 50 degrees may generally be considered characteristic or diagnostic of the crystalline form, subject however to standard practice in crystallography.

The intensities of the XRPD peaks for this crystalline form in the 2-theta range 5 to 50 degrees are shown in Table A below, in the column headed "Form V". From this data, the d-spacings may readily be calculated using the Bragg equation.

The form V material may be prepared in relatively pure form by recrystallisation of commercially available smilagenin from ethanol, tetrahydrofuran, hexane or aqueous acetone. The acetone/water proportions in the aqueous acetone may vary widely, for example from about 0.5:1 (by volume) to about 25:1 (by volume), for example from about 1:1 (by volume) to about 20:1 (by volume), for example from about 2:1 (by volume) to about 19:1 (by volume),

A solvent mediated transformation of commercially available smilagenin in butanone or in 50% aqueous ethanol yielded a mixture of crystal forms III and V.

A solvent mediated transformation of commercially available smilagenin in dichloromethane yielded a mixture of crystal forms II and V.

Solvent mediated transformations in aqueous acetone, aqueous tetrahydrofuran or aqueous ethanol all yield crystal form V (monohydrate). In each case the proportions of water to solvent in the medium can vary widely. The purity of the resultant crystalline material appears to be higher when aqueous acetone is used, in comparison with the alternative media.

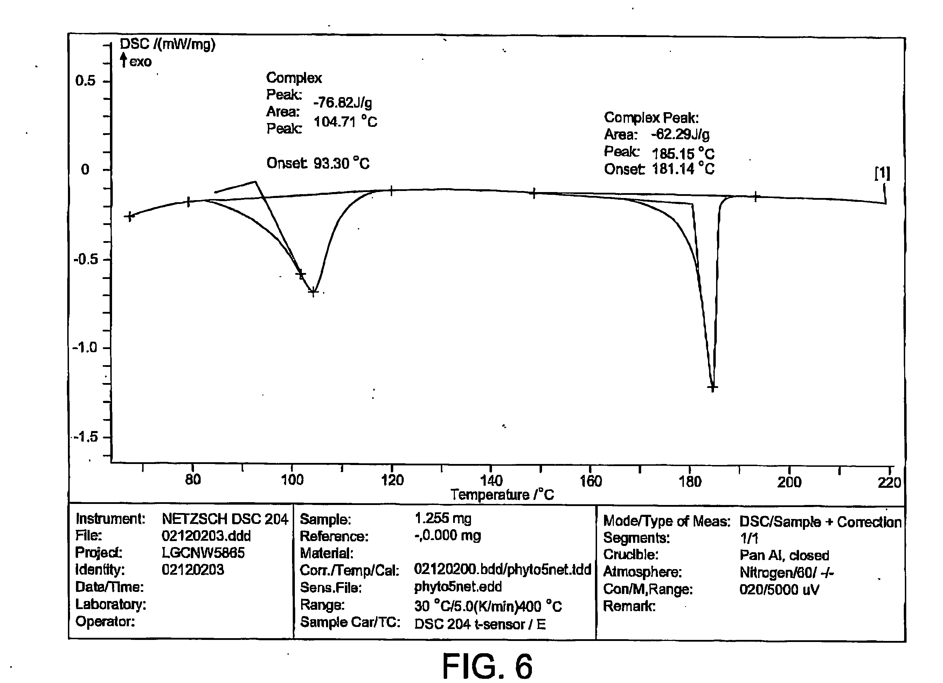

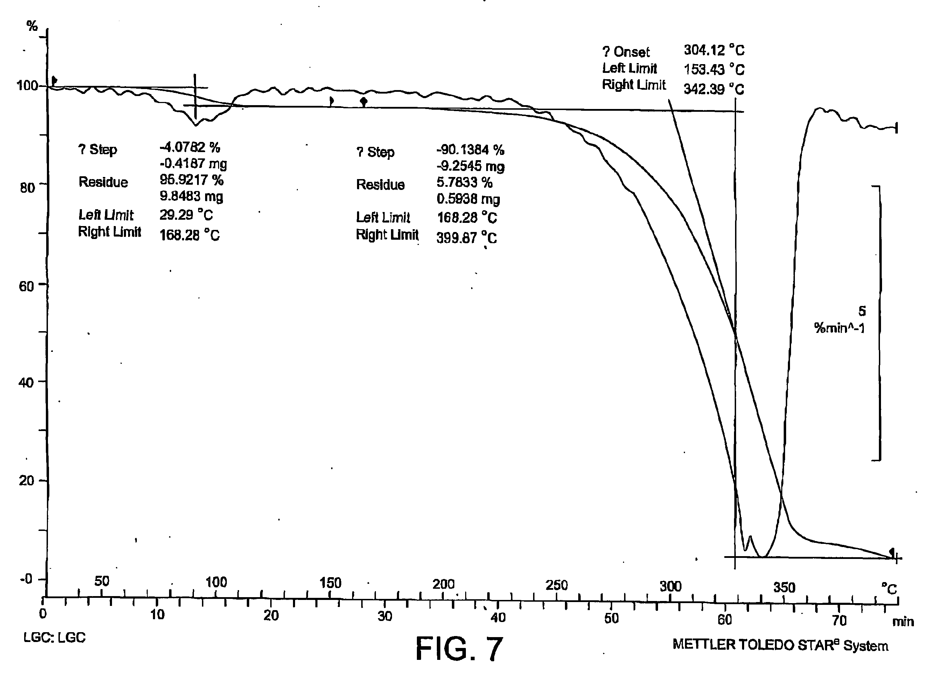

Karl Fischer analysis, DSC (Figure 6) and TGA (Figure 7) confirmed that the crystal form V that we have obtained is a monohydrate of smilagenin.

### Crystalline Form VI

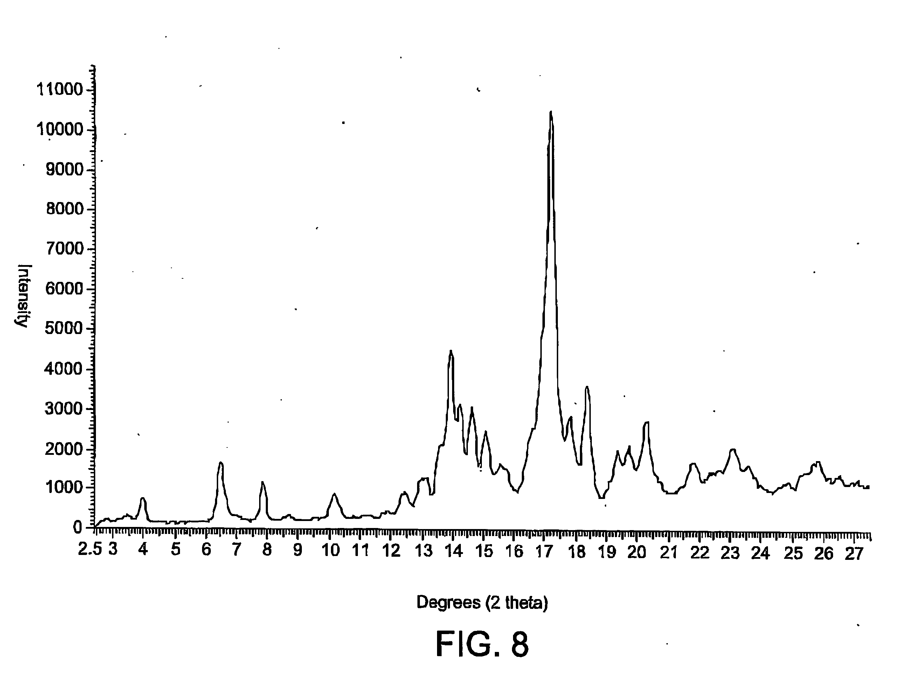

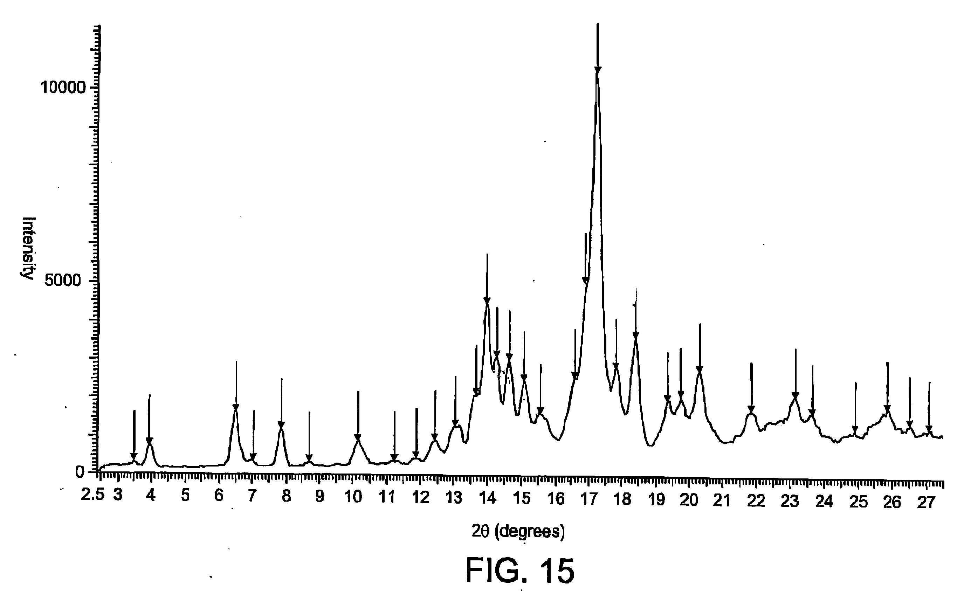

The term "crystalline form VI" used herein means that crystalline form of smilagenin which has an XRPD pattern substantially as shown in Figures 8 and 15 of the accompanying drawings (λ = 1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 2 to 28 degrees may generally be considered characteristic or diagnostic of the crystalline form, subject however to standard practice in crystallography.

The intensities of the XRPD peaks for this crystalline form in the 2-theta range 2 to 28 degrees can be obtained from Figures 8 and 15. The 2θ and d-spacings for the significant peaks, and the intensities of the significant peaks relative to the strongest peak, are given in Table B.

The form VI material may be prepared in relatively pure form by recrystallisation of commercially available smilagenin from methanol.

DSC (Figure 9), TGA (Figure 10) and vapour sorption (Figure 11) analysis suggest that the crystal form VI that we have obtained is a channel hydrate of smilagenin exhibiting variable smilagenin:water stoichiometry and the potential to form channel solvates with solvents having appropriately sized molecules. As is well known, a channel hydrate is a hydrate in which the crystal lattice of the molecule forms a channel or cage enclosing a void which can wholly or partially accommodate water (or solvent) molecules. The stoichiometric (molar) ratio of smilagenin:water at any particular time will depend on such factors as the humidity of the surrounding atmosphere, as the water molecules are generally free to enter or leave the void.

### Crystalline Form VII

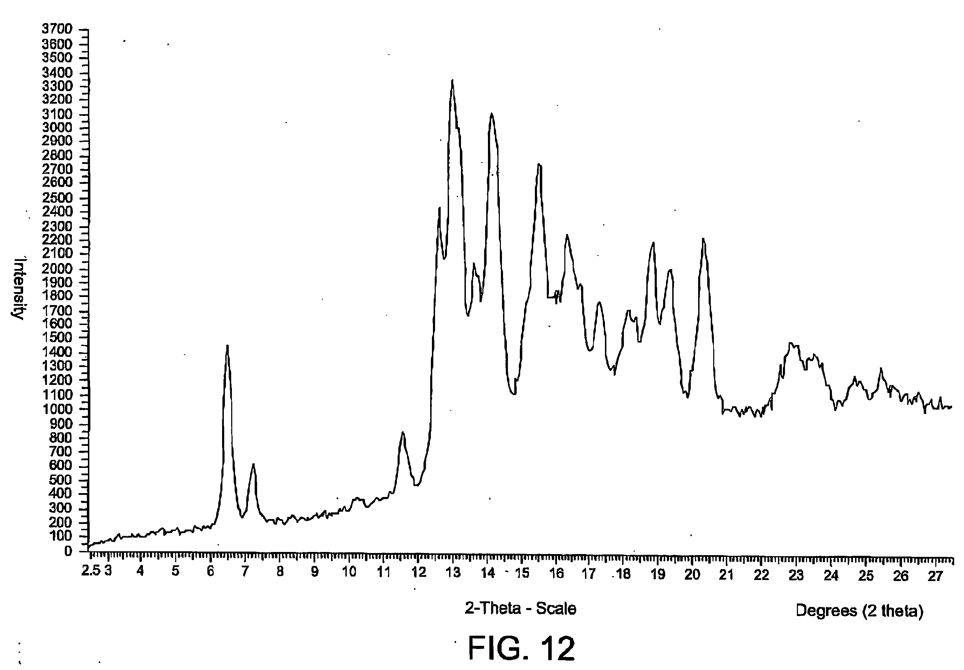

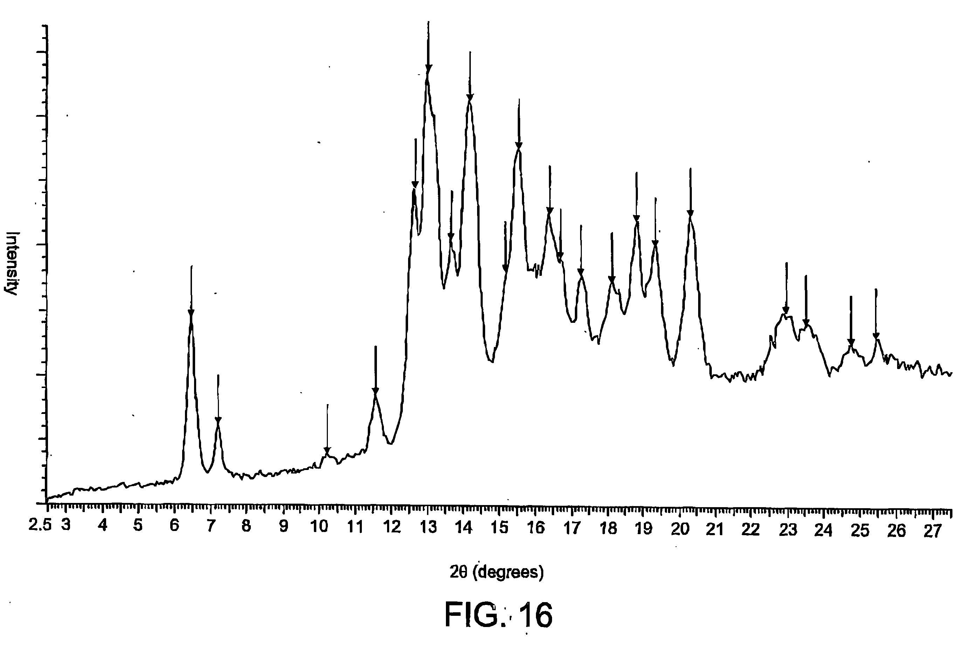

The term "crystalline form VII" used herein means that crystalline form of smilagenin which has an XRPD pattern substantially as shown in Figures 12 and 16 of the accompanying drawings (λ =1.5406 Angstroms).

The expression "an XRPD pattern substantially as shown" as used herein refers particularly to any XRPD pattern having 2-theta or d-spacing peaks corresponding to the diagnostic peaks of the Figure. For present purposes, the approximately 20 strongest peaks in the 2-theta range 2 to 28 degrees may generally be considered characteristic or diagnostic of the crystalline form, subject however to standard practice in crystallography.

The intensities of the XRPD peaks for this crystalline form in the 2-theta range 2 to 28 degrees can be obtained from Figures 12 and 16. The 2θ and d-spacings for the significant peaks, and the intensities of the significant peaks relative to the strongest peak, are given in Table C.

The form VII material may be prepared in relatively pure form by crystallisation of smilagenin from iso-propyl alcohol (IPA). The material may subsequently be aged if desired (e.g. at ambient temperature and e.g. for a period of at least about 24 hours, for example at least about 48 hours). If desired, the form VII material may be subsequently recrystallised from acetone to afford anhydrous unsolvated smilagenin in substantially pure form.

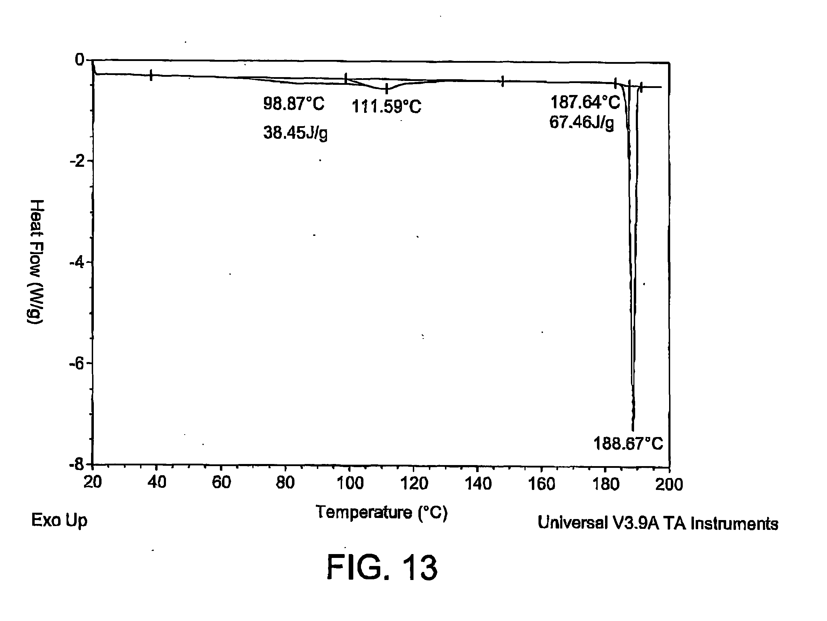

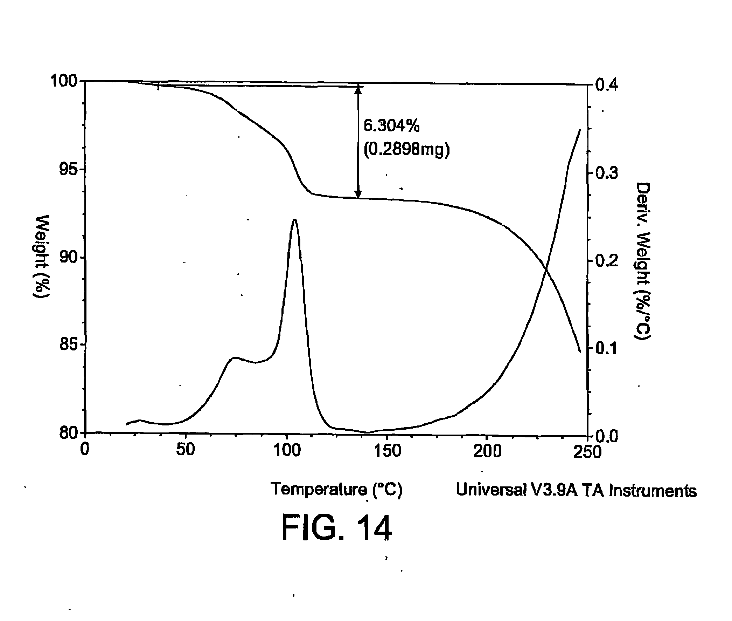

DSC (Figure 13) and TGA (Figure 14) indicate that the crystal form VII that we have obtained is a hemi-IPA solvate of smilagenin.

The form VII material is potentially important as an intermediate in the purification of smilagenin to produce pharmaceutical or edible grade smilagenin. Without wishing to be bound by theory, it is believed that a solution of relatively impure smilagenin in *iso-*propyl alcohol is especially convenient for being azeotropically distilled in order to efficiently remove water from the solution. The IPA solvate of smilagenin which is precipitated from the reduced solution after the said distillation can conveniently be recrystallised as pharmaceutical or edible grade anhydrous unsolvated smilagenin using an anhydrous IPA-compatible organic solvent such as acetone, which does not form a solvate with smilagenin.

Therefore, in one particular embodiment, the form VII material may be in substantially pure isolated, preferably dry, form and prepared by precipitation from a solution of relatively impure smilagenin in iso-propanol, which solution has preferably previously undergone distillation to reduce its volume and remove water or other impurities. Most preferably, the process by which the form VII material is made is conducted on an industrial scale (obtaining at least kilogram quantities of the form VII material). Preferably, anhydrous unsolvated smilagenin is subsequently recrystallised in pharmaceutical or edible grade from the said form VII material using an anhydrous IPA-compatible organic solvent such as acetone, which does not form a solvate with smilagenin. It is further preferred that at no stage in the preparation or purification of the smilagenin, including the stage of formation of any form VII material or other intermediate form of smilagenin, is it necessary (or done) to remove water from the smilagenin, or from any mixture containing it, using a solid hygroscopic material such as magnesium sulphate.

Examples 2, 6 and 7 of WO-A-2004/037845 describe certain laboratory scale batchwise procedures for purifying smilagenin via recrystallisation from iso-propyl alcohol (2-propanol). However, in none of these Examples is it stated that the precipitated material is an IPA solvate of smilagenin, let alone a hemi-IPA solvate. To the extent that it may be necessary in any jurisdiction to exclude the disclosures of those examples and subject-matter that is obvious therefrom from the scope of protection for the form VII material, processes for its preparation, and uses thereof, such disclosures - and especially the disclosures of the purification of the initial impure smilagenin using iso-propyl alcohol - are hereby disclaimed from the present application. In particular, in such jurisdictions the form VII material according to the present invention may comprise the form VII material in substantially pure isolated form prepared on a kilogram scale. As mentioned above, this material is precipitated from the reduced IPA solution after azeotropic distillation and can conveniently be recrystallised as pharmaceutical or edible grade anhydrous unsolvated smilagenin (e.g. in form I or form III, e.g. in substantially pure isolated form prepared on a kilogram scale) using an anhydrous IPA-compatible organic solvent such as acetone, which does not form a solvate with smilagenin.

### Amorphous Form

The amorphous form is non-crystalline. We have found that amorphous smilagenin appears to have potentially useful water-solubility and stability with respect to conversion to a crystal form. These characteristics offer improved manufacture, formulation, storage and bioavailability of smilagenin in comparison with the prior art crystalline form.

The amorphous smilagenin we have prepared has an XRPD pattern which shows no peaks that would be characteristic of any crystalline structure.

### Derivatives

The term "derivatives" used herein refers particularly to the compounds defined and described in the prior art patent documents acknowledged above in relation to the known biological activities of smilagenin (US Patent No. 3890438; US Patent No. 4680289; US Patent No. 6258386; WO-A-01/23406; WO-A-01/23407; WO-A-01/23408; WO-A-01/49703; WO-A-02/079221; and WO-A-03/082893).

Such derivatives include pharmaceutically acceptable pro-drugs of smilagenin and pharmaceutically acceptable salts thereof.

Pro-drugs of smilagenin may especially include 3-position carboxylate esters such as the cathylate (ethoxycarbonyloxy), acetate, succinate, propionate, butyrate, valerate, isovalerate, caproate, isocaproate, diethylacetate, octanoate, decanoate, laurate, myristate, palmitate, stearate, benzoate, phenylacetate, phenylpropionate, cinnamate, p-nitrobenzoyloxy, 3,5-dinitrobenzoyloxy, p-chlorobenzoyloxy, 2,4-dichlorobenzoyloxy, p-bromobenzoyloxy, m-bromobenzoyloxy, p-methoxy-benzoyloxy, phthalyl, glycinate, alaninate, valinate, phenylalaninate, isoleucinate, methioninate, argininate, aspartate, cysteinate, glutaminate, histidinate, lysinate, prolinate, serinate, threoninate, tryptophanate, tyrosinate, fumarate and maleate esters.

"Pharmaceutically acceptable salts" means the relatively non-toxic, inorganic and organic acid addition salts, and base addition salts, of compounds of the present invention. These salts can be prepared *in situ* during the final isolation and purification of the compounds. In particular, acid addition salts can be prepared by separately reacting the purified compound in its free base form with a suitable organic or inorganic acid and isolating the salt thus formed. See, for example S. M. Berge et al., Pharmaceutical Salts, J. Pharm. Sci., 66: pp.1-19 (1977) which is incorporated herein by reference. Base addition salts can also be prepared by separately reacting the purified compound in its acid form with a suitable organic or inorganic base and isolating the salt thus formed. Base addition salts include pharmaceutically acceptable metal and amine salts. Examples of suitable acid addition salts are those formed with acids selected from hydrochloric, sulphuric, phosphoric and nitric acids. Examples of suitable base addition salts are those formed with bases selected from sodium hydroxide, potassium hydroxide and ammonium hydroxide.

### Medicaments, Foodstuffs, Food Supplements and Beverages

According to the invention, a composition may comprise a material as described above in admixture with one or more further component selected from: one or more other materials as described above, another form of smilagenin, any other biologically active material, and any biologically inactive material.

The composition may be prepared by a method comprising admixing a material as described above with one or more further component selected from: one or more other materials as described above, another form of smilagenin, any other biologically active material, and any biologically inactive material.

According to the invention, the material or the composition (e.g. the medicament, foodstuff, food supplement or beverage) may be used for the treatment of a condition selected from: high blood cholesterol levels, obesity and diabetes obesity syndromes, cognitive dysfunction and allied conditions, non-cognitive neurodegeneration, non-cognitive neuromuscular degeneration, motor-sensory neurodegeneration and loss of receptor function in the absence of cognitive, neural or neuromuscular impairment.

Still further, the invention therefore provides a method of treatment of a human or non-human animal (e.g. a human) suffering from, or susceptible to, a condition selected from: high blood cholesterol levels, obesity and diabetes obesity syndromes, cognitive dysfunction and allied conditions, non-cognitive neurodegeneration, non-cognitive neuromuscular degeneration, motor-sensory neurodegeneration and loss of receptor function in the absence of cognitive, neural or neuromuscular impairment, which comprises administering to the said human or non-human animal an effective amount of a material or composition as described above.

The active agent prepared according to the present invention may thus be formulated into any suitable composition form for administration to a human or non-human animal patient. The composition may consist of the active agent alone or may include the active agent and any suitable additional component, such as one or more pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms.

The composition may, for example, be a pharmaceutical composition (medicament), a foodstuff, food supplement or beverage.

The terms "foodstuff', "food supplement" and "beverage" used herein have the normal meanings for those terms, and are not restricted to pharmaceutical preparations. The appropriate pharmaceutical or edible grade of ingredients will be used, according to the desired composition form.

For further details of suitable composition forms and dosages, please refer to US Patent No. 3890438, US Patent No. 4680289; US Patent No. 6258386; WO-A-01/23406; WO-A-01/23407; WO-A-01/23408; WO-A-01/49703; WO-A-02/07922; and WO-A-03/082893.

### Brief Description of the Drawings

In the accompanying drawings:
Figure 1 shows an XRPD pattern (λ = 1.5406 Angstroms) obtained from a sample of commercially available smilagenin in crystalline form II (prior art);
Figure 2 shows an XRPD pattern (λ = 1.5406 Angstroms) of a sample of smilagenin in crystalline form I;
Figure 3 shows an XRPD pattern (λ = 1.5406 Angstroms) of a sample of smilagenin in crystalline form III;
Figure 4 shows an XRPD pattern (λ = 1.5406 Angstroms) of a sample of smilagenin in crystalline form IIIA;
Figure 5 shows an XRPD pattern (λ = 1.5406 Angstroms) of a sample of smilagenin in crystalline form V;
Figure 6 shows a DSC trace of a sample of smilagenin in crystalline form V, shown to be a monohydrate;
Figure 7 shows a TGA of a sample of smilagenin in crystalline form V, shown to be a monohydrate;
Figure 8 shows an XRPD pattern (λ =1.5406 Angstroms) of a sample of smilagenin in crystalline form VI (believed to be smilagenin channel hydrate);

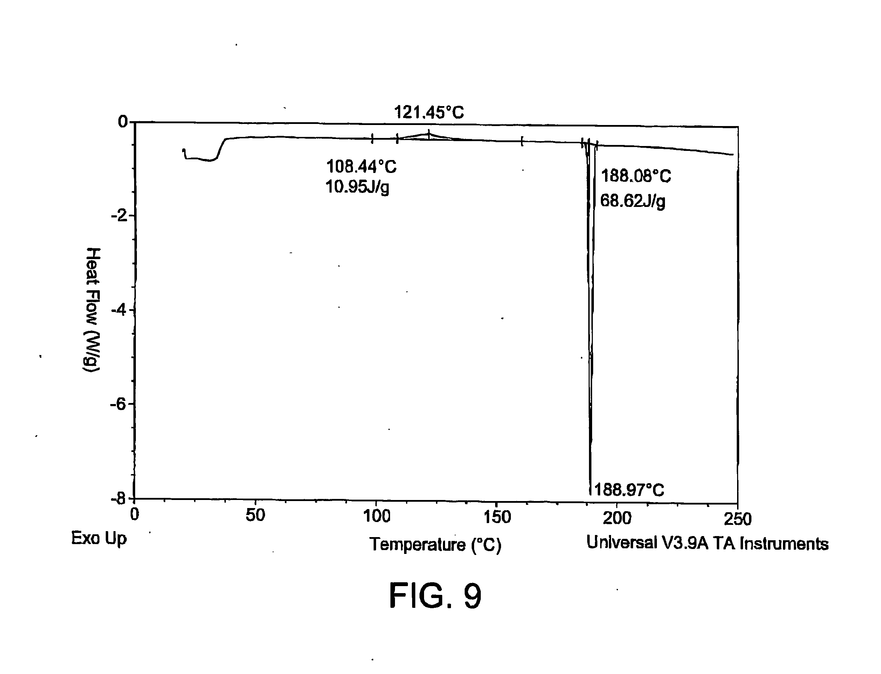

Figure 9 shows a DSC trace of a sample of smilagenin in crystalline form VI;

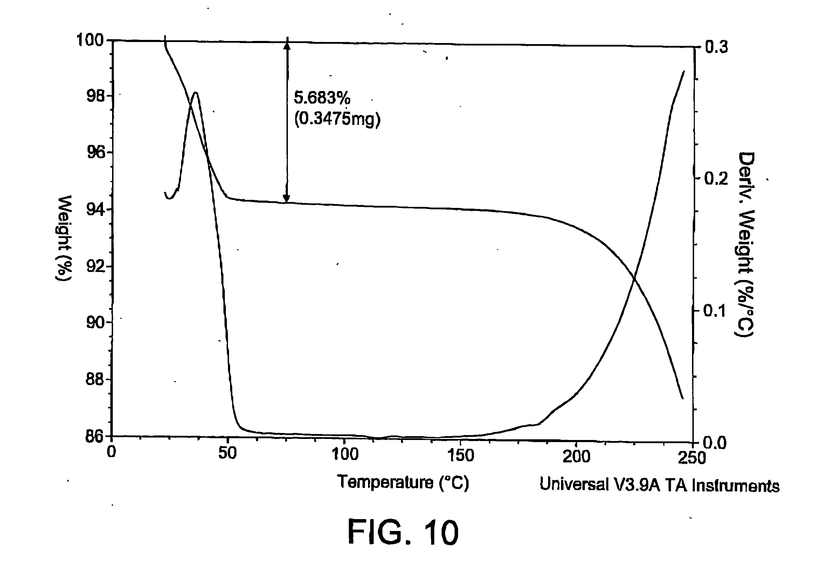

Figure 10 shows a TGA of a sample of smilagenin in crystalline form VI;

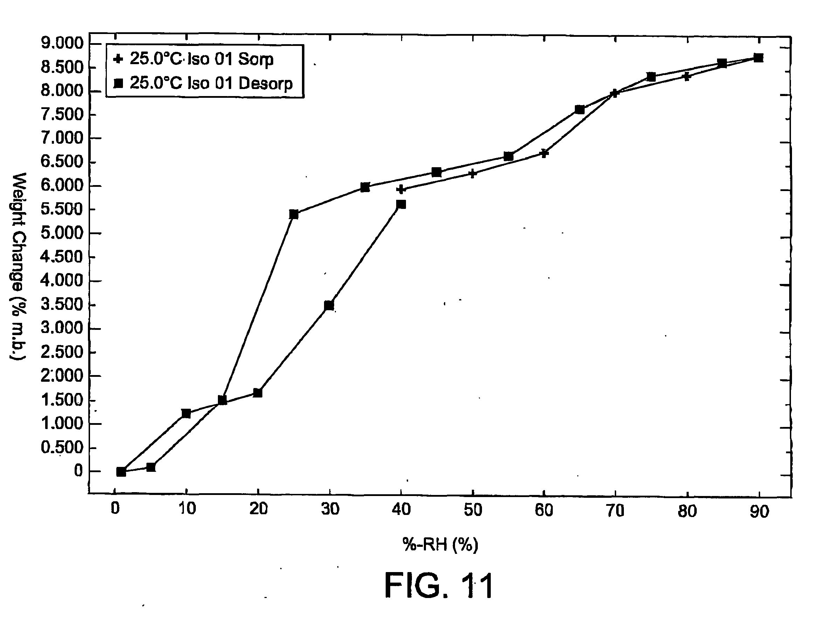

Figure 11 shows a vapour sorption graph of a sample of smilagenin in crystalline form VI;
Figure 12 shows an XRPD pattern (λ = 1.5406 Angstroms) of a sample of smilagenin **iso**-propyl alcohol (IPA)-solvate in crystalline form VII;
Figure 13 shows a DSC trace of a sample of smilagenin IPA-solvate in crystalline form VII;
Figure 14 shows a TGA of a sample of smilagenin IPA-solvate in crystalline form VII, shown to be a hemi-IPA-solvate;
Figure 15 shows an XRPD pattern (λ* = 1.5406 Angstroms) of a sample of smilagenin in crystalline form VI, with significant peaks marked by arrows; and
Figure 16 shows an XRPD pattern (λ = 1.5406 Angstroms) of a sample of smilagenin IPA-solvate in crystalline form VII, with significant peaks are marked by arrows.

### Examples and Detailed Description of the Drawings

The following non-limiting Examples are provided as further illustration of the present invention, but without limitation, and are discussed with reference to the drawings.

### Starting Materials

Samples of commercially available smilagenin were purchased from Research Plus Inc and Steraloids Inc.

The samples were analysed by XRPD and defined as form II by our nomenclature. A sample from Research Plus was examined by DSC and TGA and found to be anhydrous.

### Example 1

### Crystalline Form I

### A. Crystallisation from Acetone

Smilagenin (10.0 g) was suspended in acetone (250 ml) and the mixture heated to reflux. The resultant solution was decanted from some undissolved solids and reheated to reflux to afford a clear solution. The solution was allowed to cool over about 3.5 hours to 29°C and further cooled with an ice/water batch to 2°C. The resultant solid was harvested by filtration, washed with cold (5°C) acetone (50 ml) and dried in a vacuum oven for 3 days to afford 7.4 g of pure smilagenin, which was characterised by XRPD as form I under our nomenclature.

### B. Crystallisation from Acetonitrile

A suspension of smilagenin (1.05 g) in acetonitrile (10 ml) was stirred at ambient temperature overnight. The solid was harvested by filtration and dried in a vacuum oven at 80°C to afford smilagenin form I (0.92 g, 88% yield).

### Example 2

### Crystalline Form II

Smilagenin was manufactured by stereospecific reduction of diosgenin according to the process described in PCT Patent Application No. PCT/GB2003/001780 (WO-A-2004/037845).

The smilagenin was subjected to XRPD and the pattern was found to be substantially similar to that shown in Figure 1 of the drawings. On this basis, the material was characterised as form II under our nomenclature.

### Examples 3 and 4

### Crystalline Form III

### Example 3

Smilagenin (10.0g) was suspended in acetone (250 ml) and the mixture heated to reflux. The resultant solution was cooled to 2°C over about 15 minutes and the solid harvested by filtration, washed with cold (5°C) acetone (256 ml) and dried in a vacuum oven for about 24 hours to afford 8.1 g of pure smilagenin, which was characterised by XRPD as form III under our nomenclature.

### Example 4

Smilagenin (200 mg; form II) was suspended in tert-butyl methyl ether (4 ml) and stirred for about 48 hours. The solid was harvested by filtration and dried to afford 40 mg, which was characterised as form III under our nomenclature by XRPD, DSC and TGA.

### Examples 5 to 9

### Crystalline Form V

### Example 5

Water (200 ml) was added to a suspension of smilagenin (20 g) in acetone (200 ml) and the mixture stirred for about 2 hours. The solid was harvested by filtration, dried in a vacuum oven at 40°C for about 24 hours to afford 20.5 g which was characterised by XRPD as form V under our nomenclature. The water content was determined as 4.4% by Karl Fischer analysis.

### Example 6

Smilagenin (200 mg; form II) was suspended in hexane (10 ml) and stirred for about 48 hours. The solid was harvested by filtration and dried to afford 80 mg which was characterised as form V under our nomenclature by XRPD, DSC and TGA.

### Example 7

Smilagenin (500 mg; form II) was suspended in tetrahydrofuran (2 ml) and stirred for about 48 hours. The solid was harvested by filtration and dried to afford 80 mg which was characterised as form V under our nomenclature by XRPD, DSC and TGA.

### Examples 8 and 9

Smilagenin (Research Plus Inc.) was recrystallised using slurry crystallisation with butanone (Example 8) or 50% aqueous ethanol (Example 9) as the recrystallisation solvent. In each case the recrystallised material was subjected to XRPD and was characterised as a mixture of forms III and V under our nomenclature.

### Example 10

### Crystalline Form IIIA

Smilagenin (Research Plus Inc) was recrystallised using slurry crystallisation with dimethylformamide as the recrystallisation solvent.

The recrystallised material was subjected to XRPD and on this basis the material was characterised as crystalline form IIIA under our nomenclature.

### Example 11

### Crystalline Form VI (smilagenin channel hydrate)

Smilagenin (260 mg) was weighed into a round bottomed flask and methanol (10 ml) was added. The contents were heated to 70°C to affect complete dissolution, allowed to cool to room temperature and stirred at room temperature for 60 minutes. The solid was collected by filtration and air dried for about 2.5hours.

The X-ray powder diffraction pattern is shown in Figure 8, the differential scanning calorimetry trace is shown in Figure 9 and the thermogravimetric analysis is shown in Figure 10.

The DSC trace shows a weak broad endothermic transition up to 50°C, an exothermic transition at 120°C followed by a final high energy melting transition at 188°C. TGA analysis confirms that the initial endotherm is associated with loss of water and that the transition at 121°C is not associated with any solvent loss. Analysis of the sample by Karl Fischer titration confirmed that the solvent was water.

Vapour sorption studies (see Figure 11) show that the sample is hygroscopic and adsorbs up to 9% water (about 2 mol. eq. water; i.e. a dihydrate) at high humidity and reversibly loses the water at low humidity. This suggests that this form is a channel hydrate of variable smilagenin:water stoichiometry, depending on the surrounding temperature and humidity.

### Example 12

### Crystalline Form VII (smilagenin IPA-solvate)

Smilagenin (150 mg) was added to iso-propyl alcohol (IPA) (5 ml) and the mixture heated to 70°C to ensure dissolution. The clear solution was then allowed to cool to 40°C over 2 hours, whereupon sudden precipitation occurred. The mixture was reheated to 65°C to dissolve the material and the solution re-cooled to 45°C, held at 45°C for 15 minutes, cooled to 40°C, and held at 40°C for 2 hours. The slurry was then cooled to room temperature and aged over a weekend at room temperature. After this time the solid was collected by filtration and air dried for about 3 hours.

The X-ray powder diffraction pattern is shown in Figure 12, the differential scanning calorimetry trace is shown in Figure 13 and the thermogravimetric analysis is shown in Figure 14.

The DSC shows a broad endotherm between 40°C and 140°C that is consistent with loss of IPA from the sample. This is confirmed by TGA analysis which indicates 6.3% IPA present in the sample which is consistent with a hemi-IPA solvate of smilagenin. The IPA appears to be loosely bound in the crystal as it is lost from about 40°C upwards in the DSC.

### Example 13

### Amorphous smilagenin

Smilagenin (10 g) was heated to its melt using a temperature controlled heating mantle and held until a complete molten liquid was formed. The molten mass was poured into a Dewar containing approximately 150ml of liquid nitrogen. The sample was decanted into a glass beaker and the liquid nitrogen allowed to evaporate. The sample was then transferred to a glass vial, flushed with dry nitrogen and then sealed. The sample was characterised as amorphous smilagenin on the basis of the XRPD pattern, which showed a lack of significant diffraction lines.

### TABLE A

**XRPD Intensities for Smilagenin Crystal Forms I, II, III and V (λ = 1.5406 Angstroms) at regularly spaced intervals in the 2-theta Range 5 to 50 degrees**

| λ = 1.5406 Å Degrees (2θ) | Form I | Form II | Form III | Form V |
|---|---|---|---|---|
| 5 | 225 | 174 | 384 | 231 |
| 5.02 | 231 | 154 | 404 | 204 |
| 5.04 | 231 | 159 | 384 | 193 |
| 5.06 | 228 | 185 | 380 | 240 |
| 5.08 | 225 | 177 | 365 | 199 |
| 5.1 | 199 | 172 | 392 | 225 |
| 5.12 | 210 | 202 | 365 | 188 |
| 5.14 | 199 | 172 | 428 | 222 |
| 5.16 | 213 | 188 | 392 | 207 |
| 5.18 | 169 | 207 | 396 | 210 |
| 5.2 | 185 | 164 | 357 | 213 |
| 5.22 | 216 | 196 | 404 | 216 |
| 5.24 | 174 | 185 | 350 | 193 |
| 5.26 | 196 | 188 | 420 | 213 |
| 5.28 | 156 | 177 | 396 | 213 |
| 5.3 | 216 | 202 | 361 | 231 |
| 5.32 | 185 | 180 | 396 | 202 |
| 5.34 | 207 | 188 | 396 | 216 |
| 5.36 | 207 | 202 | 372 | 234 |
| 5.38 | 216 | 190 | 369 | 234 |
| 5.4 | 228 | 185 | 400 | 216 |
| 5.42 | 188 | 193 | 369 | 231 |
| 5.44 | 185 | 193 | 365 | 207 |
| 5.46 | 190 | 228 | 342 | 246 |
| 5.48 | 196 | 222 | 376 | 228 |
| 5.5 | 246 | 207 | 372 | 256 |
| 5.52 | 193 | 199 | 458 | 262 |
| 5.54 | 202 | 216 | 346 | 279 |
| 5.56 | 202 | 185 | 357 | 262 |
| 5.58 | 222 | 228 | 361 | 339 |
| 5.6 | 196 | 262 | 350 | 306 |
| 5.62 | 210 | 219 | 369 | 335 |
| 5.64 | 202 | 279 | 357 | 353 |
| 5.66 | 207 | 250 | 365 | 372 |
| 5.68 | 199 | 256 | 324 | 404 |
| 5.7 | 204 | 256 | 346 | 392 |
| 5.72 | 199 | 256 | 372 | 484 |
| 5.74 | 204 | 282 | 350 | 520 |
| 5.76 | 210 | 335 | 392 | 511 |
| 5.78 | 222 | 317 | 380 | 620 |
| 5.8 | 231 | 412 | 353 | 756 |
| 5.82 | 210 | 441 | 357 | 864 |
| 5.84 | 190 | 458 | 353 | 1116 |
| 5.86 | 253 | 471 | 342 | 1347 |
| 5.88 | 222 | 471 | 388 | 1529 |
| 5.9 | 193 | 372 | 342 | 1731 |
| 5.92 | 193 | 174 | 365 | 1576 |
| 5.94 | 196 | 177 | 384 | 918 |
| 5.96 | 210 | 172 | 396 | 424 |
| 5.98 | 199 | 146 | 380 | 231 |
| 6 | 202 | 161 | 365 | 243 |
| 6.02 | 193 | 146 | 369 | 185 |
| 6.04 | 213 | 185 | 400 | 177 |
| 6.06 | 196 | 149 | 392 | 174 |
| 6.08 | 216 | 139 | 424 | 159 |
| 6.1 | 216 | 149 | 428 | 159 |
| 6.12 | 272 | 169 | 449 | 154 |
| 6.14 | 240 | 128 | 462 | 174 |
| 6.16 | 219 | 237 | 467 | 180 |
| 6.18 | 210 | 151 | 506 | 182 |
| 6.2 | 250 | 164 | 502 | 154 |
| 6.22 | 289 | 156 | 529 | 154 |
| 6.24 | 262 | 149 | 552 | 154 |
| 6.26 | 246 | 137 | 543 | 154 |
| 6.28 | 272 | 154 | 424 | 156 |
| 6.3 | 269 | 128 | 380 | 177 |
| 6.32 | 272 | 174 | 342 | 185 |
| 6.34 | 259 | 164 | 313 | 164 |
| 6.36 | 310 | 117 | 369 | 146 |
| 6.38 | 313 | 137 | 324 | 128 |
| 6.4 | 331 | 159 | 320 | 161 |
| 6.42 | 384 | 137 | 339 | 146 |
| 6.44 | 365 | 137 | 350 | 156 |
| 6.46 | 384 | 144 | 299 | 135 |
| 6.48 | 437 | 151 | 339 | 151 |
| 6.5 | 467 | 151 | 335 | 146 |
| 6.52 | 543 | 137 | 317 | 139 |
| 6.54 | 590 | 137 | 328 | 166 |
| 6.56 | 600 | 144 | 361 | 146 |
| 6.58 | 751 | 142 | 372 | 159 |
| 6.6 | 864 | 142 | 335 | 142 |
| 6.62 | 986 | 156 | 328 | 154 |
| 6.64 | 1116 | 156 | 346 | 169 |
| 6.66 | 1274 | 151 | 335 | 177 |
| 6.68 | 1274 | 135 | 396 | 137 |
| 6.7 | 1190 | 146 | 388 | 159 |
| 6.72 | 1183 | 159 | 328 | 151 |
| 6.74 | 1325 | 182 | 376 | 159 |
| 6.76 | 1747 | 123 | 412 | 132 |
| 6.78 | 2070 | 132 | 380 | 151 |
| 6.8 | 2470 | 137 | 365 | 159 |
| 6.82 | 3226 | 137 | 380 | 149 |
| 6.84 | 3192 | 149 | 441 | 135 |
| 6.86 | 4122 | 144 | 424 | 130 |
| 6.88 | 6906 | 149 | 416 | 180 |
| 6.9 | 6939 | 161 | 449 | 144 |
| 6.92 | 4638 | 166 | 506 | 164 |
| 6.94 | 2162 | 151 | 520 | 159 |
| 6.96 | 718 | 139 | 515 | 161 |
| 6.98 | 342 | 161 | 576 | 169 |
| 7 | 310 | 135 | 566 | 151 |
| 7.02 | 272 | 132 | 640 | 151 |
| 7.04 | 259 | 146 | 671 | 123 |
| 7.06 | 250 | 154 | 745 | 139 |
| 7.08 | 276 | 146 | 801 | 132 |
| 7.1 | 256 | 128 | 858 | 139 |
| 7.12 | 237 | 114 | 847 | 144 |
| 7.14 | 269 | 128 | 1076 | 154 |
| 7.16 | 266 | 144 | 1082 | 159 |
| 7.18 | 259 | 137 | 1190 | 132 |
| 7.2 | 246 | 139 | 1267 | 146 |
| 7.22 | 234 | 149 | 1225 | 146 |
| 7.24 | 225 | 130 | 1043 | 156 |
| 7.26 | 213 | 137 | 740 | 144 |
| 7.28 | 185 | 128 | 534 | 154 |
| 7.3 | 174 | 142 | 400 | 139 |
| 7.32 | 164 | 146 | 369 | 164 |
| 7.34 | 180 | 114 | 380 | 172 |
| 7.36 | 166 | 151 | 313 | 169 |
| 7.38 | 164 | 128 | 342 | 146 |
| 7.4 | 172 | 142 | 286 | 123 |
| 7.42 | 169 | 135 | 313 | 139 |
| 7.44 | 146 | 154 | 299 | 154 |
| 7.46 | 169 | 142 | 289 | 139 |
| 7.48 | 164 | 144 | 296 | 149 |
| 7.5 | 164 | 117 | 306 | 159 |
| 7.52 | 151 | 128 | 286 | 156 |
| 7.54 | 166 | 142 | 299 | 169 |
| 7.56 | 154 | 154 | 303 | 144 |
| 7.58 | 144 | 130 | 299 | 119 |
| 7.6 | 128 | 156 | 292 | 144 |
| 7.62 | 154 | 156 | 289 | 154 |
| 7.64 | 164 | 117 | 286 | 139 |
| 7.66 | 146 | 144 | 269 | 144 |
| 7.68 | 137 | 121 | 256 | 144 |
| 7.7 | 159 | 169 | 286 | 161 |
| 7.72 | 149 | 144 | 269 | 142 |
| 7.74 | 161 | 164 | 266 | 166 |
| 7.76 | 132 | 125 | 266 | 139 |
| 7.78 | 151 | 112 | 282 | 130 |
| 7.8 | 161 | 130 | 289 | 149 |
| 7.82 | 156 | 128 | 269 | 151 |
| 7.84 | 142 | 161 | 299 | 139 |
| 7.86 | 166 | 128 | 272 | 149 |
| 7.88 | 135 | 139 | 276 | 146 |
| 7.9 | 139 | 142 | 269 | 142 |
| 7.92 | 128 | 144 | 266 | 159 |
| 7.94 | 132 | 130 | 256 | 172 |
| 7.96 | 210 | 144 | 276 | 161 |
| 7.98 | 128 | 146 | 279 | 151 |
| 8 | 146 | 128 | 292 | 146 |
| 8.02 | 146 | 130 | 253 | 137 |
| 8.04 | 151 | 137 | 299 | 159 |
| 8.06 | 259 | 119 | 256 | 161 |
| 8.08 | 125 | 121 | 276 | 154 |
| 8.1 | 142 | 123 | 276 | 156 |
| 8.12 | 128 | 135 | 292 | 142 |
| 8.14 | 149 | 139 | 289 | 137 |
| 8.16 | 177 | 123 | 253 | 137 |
| 8.18 | 135 | 139 | 299 | 137 |
| 8.2 | 130 | 121 | 272 | 144 |
| 8.22 | 121 | 137 | 262 | 172 |
| 8.24 | 100 | 106 | 262 | 139 |
| 8.26 | 213 | 119 | 266 | 132 |
| 8.28 | 144 | 110 | 276 | 139 |
| 8.3 | 130 | 117 | 279 | 128 |
| 8.32 | 135 | 112 | 269 | 137 |
| 8.34 | 139 | 130 | 266 | 135 |
| 8.36 | 177 | 130 | 216 | 137 |
| 8.38 | 128 | 130 | 296 | 144 |
| 8.4 | 139 | 132 | 279 | 123 |
| 8.42 | 125 | 92 | 282 | 125 |
| 8.44 | 125 | 112 | 272 | 154 |
| 8.46 | 114 | 121 | 256 | 135 |
| 8.48 | 303 | 130 | 262 | 137 |
| 8.5 | 114 | 117 | 289 | 142 |
| 8.52 | 119 | 128 | 256 | 139 |
| 8.54 | 149 | 110 | 282 | 125 |
| 8.56 | 132 | 121 | 289 | 188 |
| 8.58 | 119 | 132 | 279 | 142 |
| 8.6 | 110 | 128 | 243 | 121 |
| 8.62 | 106 | 98 | 272 | 137 |
| 8.64 | 110 | 130 | 289 | 159 |
| 8.66 | 125 | 117 | 313 | 128 |
| 8.68 | 125 | 117 | 299 | 114 |
| 8.7 | 114 | 104 | 303 | 313 |
| 8.72 | 135 | 128 | 317 | 132 |
| 8.74 | 121 | 125 | 303 | 135 |
| 8.76 | 112 | 128 | 306 | 121 |
| 8.78 | 112 | 130 | 313 | 112 |
| 8.8 | 130 | 106 | 320 | 135 |
| 8.82 | 119 | 142 | 350 | 151 |
| 8.84 | 119 | 125 | 320 | 137 |
| 8.86 | 114 | 144 | 259 | 142 |
| 8.88 | 117 | 142 | 286 | 146 |
| 8.9 | 130 | 156 | 303 | 130 |
| 8.92 | 139 | 137 | 269 | 164 |
| 8.94 | 137 | 149 | 262 | 144 |
| 8.96 | 110 | 182 | 262 | 164 |
| 8.98 | 142 | 144 | 292 | 154 |
| 9 | 119 | 151 | 282 | 137 |
| 9.02 | 135 | 154 | 269 | 166 |
| 9.04 | 130 | 159 | 262 | 207 |
| 9.06 | 130 | 161 | 286 | 169 |
| 9.08 | 149 | 188 | 279 | 199 |
| 9.1 | 137 | 188 | 303 | 202 |
| 9.12 | 144 | 199 | 313 | 210 |
| 9.14 | 130 | 210 | 306 | 225 |
| 9.16 | 130 | 210 | 292 | 222 |
| 9.18 | 144 | 216 | 286 | 219 |
| 9.2 | 149 | 253 | 303 | 250 |
| 9.22 | 142 | 222 | 313 | 225 |
| 9.24 | 149 | 276 | 313 | 276 |
| 9.26 | 164 | 310 | 328 | 269 |
| 9.28 | 193 | 335 | 310 | 303 |
| 9.3 | 182 | 369 | 357 | 342 |
| 9.32 | 182 | 392 | 392 | 361 |
| 9.34 | 188 | 441 | 380 | 433 |
| 9.36 | 228 | 511 | 400 | 388 |
| 9.38 | 213 | 543 | 408 | 449 |
| 9.4 | 246 | 529 | 462 | 502 |
| 9.42 | 250 | 625 | 428 | 557 |
| 9.44 | 213 | 635 | 506 | 625 |
| 9.46 | 210 | 600 | 480 | 600 |
| 9.48 | 199 | 562 | 562 | 605 |
| 9.5 | 219 | 372 | 557 | 586 |
| 9.52 | 210 | 250 | 635 | 471 |
| 9.54 | 202 | 172 | 595 | 384 |
| 9.56 | 207 | 125 | 620 | 222 |
| 9.58 | 180 | 108 | 493 | 168 |
| 9.6 | 182 | 125 | 454 | 128 |
| 9.62 | 130 | 100 | 376 | 146 |
| 9.64 | 149 | 94 | 282 | 135 |
| 9.66 | 137 | 121 | 299 | 130 |
| 9.68 | 121 | 112 | 259 | 128 |
| 9.7 | 135 | 104 | 282 | 98 |
| 9.72 | 119 | 100 | 286 | 119 |
| 9.74 | 125 | 108 | 228 | 117 |
| 9.76 | 123 | 92 | 272 | 119 |
| 9.78 | 123 | 96 | 292 | 137 |
| 9.8 | 108 | 108 | 250 | 98 |
| 9.82 | 110 | 102 | 276 | 106 |
| 9.84 | 119 | 83 | 286 | 108 |
| 9.86 | 125 | 79 | 276 | 98 |
| 9.88 | 123 | 137 | 292 | 106 |
| 9.9 | 112 | 98 | 256 | 108 |
| 9.92 | 114 | 102 | 262 | 106 |
| 9.94 | 121 | 88 | 272 | 106 |
| 9.96 | 123 | 86 | 262 | 112 |
| 9.98 | 119 | 98 | 279 | 123 |
| 10 | 119 | 106 | 282 | 94 |
| 10.02 | 96 | 98 | 250 | 98 |
| 10.04 | 130 | 108 | 286 | 117 |
| 10.06 | 144 | 112 | 266 | 102 |
| 10.08 | 125 | 108 | 269 | 108 |
| 10.1 | 146 | 108 | 289 | 83 |
| 10.12 | 125 | 102 | 256 | 125 |
| 10.14 | 146 | 114 | 292 | 106 |
| 10.16 | 132 | 121 | 289 | 104 |
| 10.18 | 142 | 108 | 286 | 114 |
| 10.2 | 156 | 94 | 282 | 125 |
| 10.22 | 164 | 130 | 269 | 123 |
| 10.24 | 169 | 104 | 256 | 98 |
| 10.26 | 154 | 102 | 286 | 114 |
| 10.28 | 182 | 100 | 286 | 110 |
| 10.3 | 172 | 102 | 292 | 125 |
| 10.32 | 161 | 90 | 286 | 94 |
| 10.34 | 172 | 79 | 269 | 102 |
| 10.36 | 204 | 313 | 276 | 102 |
| 10.38 | 219 | 164 | 266 | 94 |
| 10.4 | 240 | 96 | 289 | 96 |
| 10.42 | 225 | 92 | 306 | 100 |
| 10.44 | 188 | 90 | 292 | 110 |
| 10.46 | 219 | 81 | 306 | 119 |
| 10.48 | 234 | 86 | 262 | 106 |
| 10.5 | 266 | 79 | 292 | 98 |
| 10.52 | 279 | 100 | 272 | 106 |
| 10.54 | 320 | 88 | 317 | 108 |
| 10.56 | 346 | 108 | 262 | 96 |
| 10.58 | 353 | 100 | 303 | 106 |
| 10.6 | 428 | 76 | 303 | 112 |
| 10.62 | 590 | 108 | 324 | 112 |
| 10.64 | 812 | 102 | 372 | 119 |
| 10.66 | 888 | 100 | 303 | 100 |
| 10.68 | 745 | 96 | 286 | 100 |
| 10.7 | 681 | 92 | 289 | 110 |
| 10.72 | 671 | 100 | 292 | 98 |
| 10.74 | 818 | 108 | 299 | 117 |
| 10.76 | 1050 | 102 | 339 | 108 |
| 10.78 | 1118 | 112 | 353 | 125 |
| 10.8 | 1005 | 96 | 357 | 137 |
| 10.82 | 853 | 92 | 384 | 144 |
| 10.84 | 812 | 110 | 437 | 132 |
| 10.86 | 484 | 94 | 376 | 139 |
| 10.88 | 328 | 96 | 420 | 104 |
| 10.9 | 250 | 121 | 437 | 161 |
| 10.92 | 256 | 102 | 433 | 154 |
| 10.94 | 289 | 121 | 408 | 144 |
| 10.96 | 269 | 135 | 458 | 172 |
| 10.98 | 335 | 132 | 506 | 172 |
| 11 | 339 | 142 | 552 | 144 |
| 11.02 | 372 | 137 | 515 | 185 |
| 11.04 | 303 | 161 | 586 | 207 |
| 11.06 | 213 | 135 | 640 | 190 |
| 11.08 | 144 | 185 | 666 | 240 |
| 11.1 | 128 | 177 | 708 | 234 |
| 11.12 | 202 | 188 | 708 | 306 |
| 11.14 | 112 | 199 | 713 | 292 |
| 11.16 | 119 | 228 | 724 | 328 |
| 11.18 | 110 | 199 | 676 | 317 |
| 11.2 | 119 | 237 | 620 | 396 |
| 11.22 | 125 | 253 | 557 | 408 |
| 11.24 | 112 | 225 | 467 | 400 |
| 11.26 | 132 | 204 | 420 | 433 |
| 11.28 | 130 | 204 | 365 | 445 |
| 11.3 | 130 | 231 | 365 | 441 |
| 11.32 | 132 | 237 | 324 | 350 |
| 11.34 | 123 | 207 | 328 | 433 |
| 11.36 | 125 | 276 | 324 | 441 |
| 11.38 | 139 | 246 | 289 | 493 |
| 11.4 | 139 | 310 | 320 | 506 |
| 11.42 | 149 | 276 | 296 | 610 |
| 11.44 | 146 | 346 | 342 | 645 |
| 11.46 | 154 | 350 | 310 | 708 |
| 11.48 | 139 | 372 | 331 | 824 |
| 11.5 | 169 | 445 | 335 | 955 |
| 11.52 | 161 | 488 | 306 | 1089 |
| 11.54 | 207 | 524 | 335 | 1225 |
| 11.56 | 204 | 610 | 335 | 1414 |
| 11.58 | 207 | 702 | 313 | 1544 |
| 11.6 | 237 | 756 | 324 | 1840 |
| 11.62 | 225 | 847 | 310 | 2200 |
| 11.64 | 296 | 992 | 313 | 2694 |
| 11.66 | 328 | 1239 | 328 | 3434 |
| 11.68 | 335 | 1521 | 335 | 3844 |
| 11.7 | 250 | 1962 | 342 | 4173 |
| 11.72 | 256 | 2275 | 365 | 4651 |
| 11.74 | 188 | 1656 | 369 | 6147 |
| 11.76 | 161 | 870 | 420 | 7006 |
| 11.78 | 121 | 369 | 392 | 5227 |
| 11.8 | 149 | 193 | 384 | 2714 |
| 11.82 | 154 | 137 | 388 | 992 |
| 11.84 | 151 | 104 | 404 | 441 |
| 11.86 | 154 | 123 | 437 | 299 |
| 11.88 | 139 | 114 | 445 | 234 |
| 11.9 | 144 | 112 | 471 | 169 |
| 11.92 | 142 | 86 | 511 | 182 |
| 11.94 | 149 | 121 | 511 | 154 |
| 11.96 | 177 | 102 | 471 | 164 |
| 11.98 | 159 | 108 | 538 | 146 |
| 12 | 169 | 102 | 566 | 123 |
| 12.02 | 132 | 88 | 511 | 142 |
| 12.04 | 169 | 102 | 615 | 114 |
| 12.06 | 180 | 96 | 676 | 104 |
| 12.08 | 190 | 106 | 676 | 98 |
| 12.1 | 182 | 110 | 702 | 108 |
| 12.12 | 166 | 77 | 801 | 100 |
| 12.14 | 182 | 94 | 824 | 106 |
| 12.16 | 210 | 83 | 900 | 104 |
| 12.18 | 250 | 83 | 1018 | 104 |
| 12.2 | 231 | 79 | 1082 | 117 |
| 12.22 | 279 | 83 | 1204 | 100 |
| 12.24 | 269 | 92 | 1246 | 98 |
| 12.26 | 259 | 74 | 1362 | 92 |
| 12.28 | 324 | 77 | 1429 | 100 |
| 12.3 | 353 | 102 | 1560 | 98 |
| 12.32 | 376 | 98 | 1632 | 110 |
| 12.34 | 445 | 85 | 1714 | 108 |
| 12.36 | 428 | 92 | 1689 | 94 |
| 12.38 | 462 | 79 | 1722 | 108 |
| 12.4 | 420 | 98 | 1624 | 100 |
| 12.42 | 437 | 100 | 1592 | 108 |
| 12.44 | 497 | 92 | 1513 | 100 |
| 12.46 | 562 | 83 | 1296 | 104 |
| 12.48 | 676 | 90 | 1190 | 110 |
| 12.5 | 615 | 94 | 1096 | 90 |
| 12.52 | 635 | 110 | 1037 | 94 |
| 12.54 | 557 | 102 | 1024 | 102 |
| 12.56 | 497 | 102 | 986 | 123 |
| 12.58 | 488 | 117 | 980 | 128 |
| 12.6 | 493 | 98 | 924 | 119 |
| 12.62 | 497 | 108 | 936 | 106 |
| 12.64 | 400 | 108 | 1037 | 130 |
| 12.66 | 339 | 102 | 992 | 108 |
| 12.68 | 289 | 108 | 1050 | 144 |
| 12.7 | 262 | 92 | 1204 | 117 |
| 12.72 | 272 | 108 | 1260 | 256 |
| 12.74 | 303 | 102 | 1376 | 132 |
| 12.76 | 313 | 119 | 1513 | 121 |
| 12.78 | 328 | 132 | 1656 | 110 |
| 12.8 | 380 | 114 | 1747 | 98 |
| 12.82 | 353 | 135 | 1849 | 125 |
| 12.84 | 396 | 142 | 2153 | 121 |
| 12.86 | 388 | 117 | 2352 | 128 |
| 12.88 | 408 | 144 | 2570 | 121 |
| 12.9 | 562 | 128 | 2809 | 123 |
| 12.92 | 467 | 114 | 3025 | 128 |
| 12.94 | 529 | 146 | 3387 | 121 |
| 12.96 | 581 | 130 | 3516 | 114 |
| 12.98 | 534 | 154 | 3906 | 137 |
| 13 | 630 | 149 | 4238 | 135 |
| 13.02 | 581 | 142 | 4225 | 137 |
| 13.04 | 640 | 142 | 3881 | 151 |
| 13.06 | 610 | 166 | 3469 | 135 |
| 13.08 | 671 | 190 | 2894 | 132 |
| 13.1 | 660 | 146 | 2228 | 151 |
| 13.12 | 681 | 190 | 1927 | 172 |
| 13.14 | 718 | 199 | 1875 | 156 |
| 13.16 | 773 | 219 | 1823 | 164 |
| 13.18 | 894 | 213 | 1875 | 182 |
| 13.2 | 999 | 262 | 1998 | 222 |
| 13.22 | 1163 | 306 | 2237 | 262 |
| 13.24 | 1239 | 276 | 2343 | 228 |
| 13.26 | 1318 | 328 | 2530 | 228 |
| 13.28 | 1362 | 331 | 2725 | 228 |
| 13.3 | 1529 | 369 | 2841 | 240 |
| 13.32 | 1673 | 396 | 3069 | 262 |
| 13.34 | 1789 | 424 | 3329 | 279 |
| 13.36 | 1840 | 502 | 3457 | 282 |
| 13.38 | 2034 | 524 | 3672 | 292 |
| 13.4 | 2285 | 524 | 3697 | 350 |
| 13.42 | 2381 | 605 | 3528 | 376 |
| 13.44 | 2460 | 605 | 3493 | 396 |
| 13.46 | 2560 | 713 | 3505 | 380 |
| 13.48 | 2704 | 650 | 3341 | 424 |
| 13.5 | 2560 | 708 | 3341 | 462 |
| 13.52 | 2490 | 697 | 3457 | 467 |
| 13.54 | 2034 | 724 | 3446 | 529 |
| 13.56 | 1989 | 835 | 3318 | 548 |
| 13.58 | 2088 | 778 | 3181 | 581 |
| 13.6 | 2304 | 801 | 2884 | 590 |
| 13.62 | 2746 | 807 | 2520 | 655 |
| 13.64 | 3457 | 686 | 2247 | 610 |
| 13.66 | 3672 | 581 | 2162 | 576 |
| 13.68 | 3469 | 445 | 2125 | 497 |
| 13.7 | 3612 | 433 | 2247 | 416 |
| 13.72 | 3807 | 428 | 2294 | 392 |
| 13.74 | 4436 | 433 | 2372 | 320 |
| 13.76 | 5580 | 437 | 2470 | 324 |
| 13.78 | 7362 | 441 | 2621 | 317 |
| 13.8 | 10547 | 557 | 2756 | 331 |
| 13.82 | 10424 | 534 | 2798 | 361 |
| 13.84 | 7762 | 620 | 2841 | 380 |
| 13.86 | 4900 | 751 | 2809 | 357 |
| 13.88 | 2172 | 807 | 2714 | 458 |
| 13.9 | 1176 | 930 | 2601 | 511 |
| 13.92 | 778 | 1018 | 2314 | 562 |
| 13.94 | 650 | 1176 | 1945 | 610 |
| 13.96 | 718 | 1376 | 1665 | 729 |
| 13.98 | 702 | 1537 | 1296 | 734 |
| 14 | 745 | 1884 | 1176 | 900 |
| 14.02 | 724 | 2181 | 1037 | 949 |
| 14.04 | 740 | 2372 | 1011 | 1018 |
| 14.06 | 773 | 2673 | 1011 | 1163 |
| 14.08 | 894 | 3036 | 1043 | 1354 |
| 14.1 | 876 | 3411 | 1043 | 1490 |
| 14.12 | 936 | 3721 | 1211 | 1632 |
| 14.14 | 1024 | 4032 | 1218 | 1781 |
| 14.16 | 1082 | 4624 | 1318 | 2007 |
| 14.18 | 1076 | 4679 | 1421 | 2209 |
| 14.2 | 1170 | 5170 | 1490 | 2304 |
| 14.22 | 1163 | 5213 | 1764 | 2591 |
| 14.24 | 1142 | 5098 | 1772 | 2611 |
| 14.26 | 1050 | 4665 | 1892 | 2735 |
| 14.28 | 912 | 4199 | 2079 | 2735 |
| 14.3 | 724 | 3192 | 2266 | 2440 |
| 14.32 | 625 | 2098 | 2352 | 2061 |
| 14.34 | 497 | 1318 | 2500 | 1640 |
| 14.36 | 449 | 818 | 2746 | 1354 |
| 14.38 | 433 | 595 | 2884 | 1109 |
| 14.4 | 441 | 590 | 3025 | 818 |
| 14.42 | 484 | 600 | 3170 | 745 |
| 14.44 | 408 | 605 | 3283 | 778 |
| 14.46 | 480 | 702 | 3204 | 858 |
| 14.48 | 408 | 650 | 3047 | 930 |
| 14.5 | 404 | 740 | 2530 | 1109 |
| 14.52 | 445 | 734 | 2088 | 1102 |
| 14.54 | 376 | 784 | 1697 | 1183 |
| 14.56 | 339 | 876 | 1384 | 1310 |
| 14.58 | 396 | 924 | 1318 | 1399 |
| 14.6 | 372 | 906 | 1310 | 1490 |
| 14.62 | 328 | 858 | 1289 | 1482 |
| 14.64 | 328 | 681 | 1296 | 1296 |
| 14.66 | 250 | 471 | 1354 | 1089 |
| 14.68 | 286 | 331 | 1325 | 795 |
| 14.7 | 299 | 256 | 1436 | 571 |
| 14.72 | 310 | 279 | 1482 | 350 |
| 14.74 | 306 | 228 | 1600 | 256 |
| 14.76 | 292 | 243 | 1697 | 199 |
| 14.78 | 296 | 262 | 1731 | 188 |
| 14.8 | 331 | 272 | 1927 | 204 |
| 14.82 | 339 | 306 | 1989 | 222 |
| 14.84 | 369 | 317 | 2125 | 204 |
| 14.86 | 388 | 380 | 2352 | 240 |
| 14.88 | 449 | 412 | 2460 | 262 |
| 14.9 | 484 | 408 | 2601 | 262 |
| 14.92 | 571 | 420 | 2756 | 279 |
| 14.94 | 605 | 449 | 2970 | 262 |
| 14.96 | 713 | 449 | 3181 | 289 |
| 14.98 | 740 | 502 | 3283 | 328 |
| 15 | 900 | 511 | 3226 | 339 |
| 15.02 | 992 | 497 | 3260 | 342 |
| 15.04 | 1043 | 488 | 3329 | 320 |
| 15.06 | 1089 | 484 | 3552 | 353 |
| 15.08 | 1156 | 484 | 3493 | 365 |
| 15.1 | 1267 | 471 | 3624 | 372 |
| 15.12 | 1444 | 484 | 3931 | 380 |
| 15.14 | 1632 | 538 | 4147 | 408 |
| 15.16 | 1537 | 511 | 4083 | 392 |
| 15.18 | 1592 | 524 | 4382 | 433 |
| 15.2 | 1697 | 484 | 4436 | 369 |
| 15.22 | 1608 | 493 | 4382 | 404 |
| 15.24 | 1498 | 475 | 4122 | 342 |
| 15.26 | 1340 | 493 | 3931 | 350 |
| 15.28 | 1102 | 515 | 3457 | 420 |
| 15.3 | 918 | 600 | 2767 | 400 |
| 15.32 | 697 | 615 | 2362 | 437 |
| 15.34 | 660 | 724 | 1971 | 449 |
| 15.38 | 566 | 795 | 1731 | 506 |
| 15.38 | 600 | 882 | 1706 | 571 |
| 15.4 | 586 | 1063 | 1537 | 635 |
| 15.42 | 635 | 1183 | 1544 | 708 |
| 15.44 | 702 | 1362 | 1584 | 930 |
| 15.46 | 790 | 1467 | 1444 | 1018 |
| 15.48 | 807 | 1616 | 1399 | 1089 |
| 15.5 | 876 | 1884 | 1362 | 1211 |
| 15.52 | 882 | 2162 | 1369 | 1310 |
| 15.54 | 942 | 2266 | 1406 | 1391 |
| 15.58 | 1063 | 2460 | 1362 | 1616 |
| 15.58 | 1142 | 2735 | 1310 | 1858 |
| 15.6 | 1267 | 3025 | 1406 | 1936 |
| 15.62 | 1332 | 3125 | 1421 | 2190 |
| 15.64 | 1310 | 3238 | 1444 | 2352 |
| 15.66 | 1282 | 3295 | 1498 | 2470 |
| 15.68 | 1197 | 3249 | 1467 | 2570 |
| 15.7 | 1050 | 3114 | 1560 | 2540 |
| 15.72 | 1024 | 3058 | 1560 | 2550 |
| 15.74 | 1109 | 3014 | 1656 | 2652 |
| 15.76 | 1030 | 3114 | 1673 | 2809 |
| 15.78 | 1089 | 3047 | 1632 | 3102 |
| 15.8 | 1030 | 2894 | 1608 | 3283 |
| 15.82 | 1037 | 2520 | 1537 | 3014 |
| 15.84 | 961 | 1884 | 1436 | 2611 |
| 15.86 | 949 | 1225 | 1475 | 2116 |
| 15.88 | 973 | 734 | 1498 | 1537 |
| 15.9 | 942 | 400 | 1568 | 1083 |
| 15.92 | 894 | 259 | 1616 | 681 |
| 15.94 | 1030 | 219 | 1722 | 392 |
| 15.96 | 1050 | 199 | 1789 | 246 |
| 15.98 | 1096 | 161 | 1849 | 193 |
| 16 | 1037 | 185 | 1910 | 199 |
| 16.02 | 894 | 159 | 1962 | 169 |
| 16.04 | 692 | 164 | 2025 | 164 |
| 16.06 | 502 | 144 | 2007 | 196 |
| 16.08 | 416 | 130 | 1901 | 154 |
| 16.1 | 350 | 144 | 1849 | 174 |
| 16.12 | 310 | 137 | 1600 | 132 |
| 18.14 | 256 | 137 | 1544 | 151 |
| 16.16 | 269 | 121 | 1325 | 216 |
| 16.18 | 237 | 146 | 1197 | 130 |
| 16.2 | 269 | 151 | 1122 | 135 |
| 16.22 | 259 | 151 | 1136 | 149 |
| 16.24 | 256 | 161 | 1142 | 137 |
| 16.26 | 243 | 161 | 1260 | 144 |
| 16.28 | 303 | 151 | 1232 | 159 |
| 16.3 | 282 | 199 | 1414 | 161 |
| 16.32 | 313 | 188 | 1505 | 151 |
| 16.34 | 328 | 174 | 1592 | 177 |
| 18.36 | 320 | 234 | 1714 | 213 |
| 16.38 | 331 | 262 | 1875 | 202 |
| 16.4 | 361 | 299 | 1989 | 219 |
| 16.42 | 384 | 303 | 2200 | 210 |
| 16.44 | 361 | 292 | 2372 | 240 |
| 16.46 | 376 | 339 | 2725 | 266 |
| 16.48 | 420 | 342 | 2767 | 306 |
| 16.5 | 400 | 365 | 2992 | 313 |
| 16.52 | 449 | 396 | 3238 | 331 |
| 16.54 | 424 | 428 | 3422 | 342 |
| 16.56 | 471 | 428 | 3636 | 342 |
| 16.58 | 420 | 475 | 3697 | 365 |
| 16.6 | 424 | 428 | 3576 | 384 |
| 16.62 | 353 | 488 | 3295 | 412 |
| 16.64 | 339 | 529 | 2873 | 412 |
| 18.86 | 353 | 548 | 2673 | 408 |
| 16.68 | 339 | 543 | 2611 | 428 |
| 16.7 | 328 | 543 | 2652 | 437 |
| 16.72 | 313 | 557 | 2500 | 467 |
| 16.74 | 292 | 502 | 2500 | 441 |
| 16.76 | 317 | 449 | 2632 | 416 |
| 16.78 | 313 | 538 | 2683 | 380 |
| 16.8 | 296 | 529 | 2510 | 369 |
| 16.82 | 350 | 566 | 2530 | 396 |
| 16.84 | 350 | 605 | 2343 | 384 |
| 16.86 | 331 | 660 | 2070 | 404 |
| 18.88 | 369 | 671 | 1798 | 462 |
| 16.9 | 420 | 734 | 1459 | 538 |
| 16.92 | 420 | 762 | 1253 | 600 |
| 16.94 | 416 | 795 | 1129 | 590 |
| 16.96 | 454 | 882 | 1056 | 702 |
| 16.98 | 493 | 980 | 1030 | 729 |
| 17 | 538 | 1050 | 906 | 888 |
| 17.02 | 557 | 1274 | 949 | 955 |
| 17.04 | 548 | 1310 | 942 | 1056 |
| 17.06 | 571 | 1537 | 955 | 1197 |
| 17.08 | 620 | 1608 | 980 | 1303 |
| 17.1 | 630 | 1892 | 930 | 1459 |
| 17.12 | 671 | 2016 | 986 | 1568 |
| 17.14 | 801 | 2190 | 1050 | 1764 |
| 17.16 | 847 | 2480 | 1030 | 1971 |
| 17.18 | 900 | 2560 | 1043 | 2134 |
| 17.2 | 912 | 2673 | 1102 | 2381 |
| 17.22 | 967 | 2938 | 1102 | 2735 |
| 17.24 | 1030 | 2873 | 1129 | 2809 |
| 17.26 | 999 | 2894 | 1082 | 2916 |
| 17.28 | 1005 | 2591 | 1122 | 2809 |
| 17.3 | 1050 | 2088 | 1082 | 2470 |
| 17.32 | 942 | 1421 | 986 | 2285 |
| 17.34 | 955 | 858 | 1011 | 1927 |
| 17.36 | 949 | 534 | 1018 | 1429 |
| 17.38 | 1037 | 353 | 961 | 853 |
| 17.4 | 1218 | 269 | 924 | 590 |
| 17.42 | 1482 | 210 | 918 | 380 |
| 17.44 | 1781 | 185 | 999 | 250 |
| 17.46 | 1980 | 154 | 955 | 250 |
| 17.48 | 1962 | 169 | 1058 | 253 |
| 17.5 | 2043 | 169 | 1030 | 240 |
| 17.52 | 1989 | 169 | 1037 | 286 |
| 17.54 | 1868 | 174 | 1018 | 276 |
| 17.56 | 2162 | 219 | 1122 | 313 |
| 17.58 | 2218 | 219 | 1142 | 286 |
| 17.6 | 2088 | 240 | 1190 | 320 |
| 17.62 | 2275 | 190 | 1190 | 388 |
| 17.84 | 2694 | 174 | 1183 | 433 |
| 17.66 | 2611 | 144 | 1082 | 376 |
| 17.68 | 2430 | 154 | 1037 | 272 |
| 17.7 | 2323 | 123 | 999 | 253 |
| 17.72 | 2266 | 128 | 930 | 180 |
| 17.74 | 2266 | 128 | 942 | 119 |
| 17.7B | 2007 | 161 | 973 | 121 |
| 17.78 | 1927 | 117 | 918 | 149 |
| 17.8 | 1163 | 135 | 936 | 135 |
| 17.82 | 829 | 132 | 1018 | 149 |
| 17.84 | 506 | 139 | 1063 | 137 |
| 17.86 | 396 | 139 | 1136 | 154 |
| 17.88 | 376 | 180 | 1218 | 151 |
| 17.9 | 380 | 190 | 1332 | 137 |
| 17.92 | 493 | 185 | 1406 | 159 |
| 17.94 | 400 | 180 | 1444 | 190 |
| 17.96 | 376 | 182 | 1568 | 159 |
| 17.98 | 412 | 174 | 1640 | 204 |
| 18 | 412 | 164 | 1756 | 196 |
| 18.02 | 428 | 210 | 1866 | 180 |
| 18.04 | 433 | 286 | 2043 | 190 |
| 18.06 | 396 | 216 | 2034 | 216 |
| 18.08 | 353 | 196 | 2079 | 222 |
| 18.1 | 372 | 222 | 2052 | 213 |
| 18.12 | 320 | 182 | 1823 | 213 |
| 18.14 | 328 | 172 | 1640 | 202 |
| 18.16 | 246 | 154 | 1414 | 196 |
| 18.18 | 234 | 137 | 1156 | 149 |
| 18.2 | 246 | 132 | 936 | 177 |
| 18.22 | 222 | 151 | 801 | 146 |
| 18.24 | 237 | 130 | 740 | 159 |
| 18.26 | 216 | 121 | 630 | 164 |
| 18.28 | 253 | 144 | 625 | 164 |
| 18.3 | 262 | 154 | 640 | 182 |
| 18.32 | 213 | 180 | 655 | 161 |
| 18.34 | 225 | 166 | 671 | 199 |
| 18.36 | 228 | 188 | 692 | 207 |
| 18.38 | 225 | 185 | 692 | 225 |
| 18.4 | 272 | 185 | 745 | 246 |
| 18.42 | 256 | 253 | 745 | 276 |
| 18.44 | 269 | 250 | 778 | 269 |
| 18.46 | 250 | 282 | 745 | 320 |
| 18.48 | 269 | 331 | 829 | 324 |
| 18.5 | 317 | 372 | 876 | 388 |
| 18.52 | 282 | 392 | 900 | 408 |
| 18.54 | 339 | 480 | 924 | 420 |
| 18.56 | 369 | 502 | 961 | 515 |
| 18.58 | 369 | 562 | 924 | 581 |
| 18.6 | 404 | 615 | 1024 | 660 |
| 18.62 | 428 | 702 | 1037 | 713 |
| 18.84 | 441 | 724 | 1011 | 784 |
| 18.66 | 506 | 778 | 942 | 864 |
| 18.68 | 520 | 790 | 924 | 980 |
| 18.7 | 581 | 936 | 870 | 1156 |
| 18.72 | 620 | 980 | 795 | 1142 |
| 18.74 | 734 | 1005 | 724 | 1260 |
| 18.76 | 724 | 967 | 666 | 1176 |
| 18.78 | 692 | 992 | 605 | 1176 |
| 18.8 | 790 | 955 | 635 | 1136 |
| 18.82 | 864 | 864 | 576 | 1122 |
| 18.84 | 900 | 835 | 534 | 1063 |
| 18.86 | 900 | 818 | 529 | 961 |
| 18.88 | 900 | 692 | 538 | 870 |
| 18.9 | 847 | 543 | 543 | 762 |
| 18.92 | 858 | 376 | 511 | 620 |
| 18.94 | 853 | 289 | 529 | 497 |
| 18.96 | 864 | 369 | 534 | 369 |
| 18.98 | 900 | 166 | 557 | 231 |
| 19 | 864 | 130 | 586 | 196 |
| 19.02 | 912 | 112 | 586 | 144 |
| 19.04 | 1122 | 117 | 586 | 130 |
| 19.06 | 1282 | 90 | 595 | 132 |
| 19.08 | 1429 | 98 | 640 | 102 |
| 19.1 | 1436 | 104 | 640 | 112 |
| 19.12 | 1310 | 128 | 650 | 100 |
| 19.14 | 1218 | 98 | 671 | 130 |
| 19.16 | 1190 | 112 | 676 | 114 |
| 19.18 | 1296 | 85 | 686 | 106 |
| 19.2 | 1616 | 104 | 666 | 119 |
| 19.22 | 1475 | 112 | 655 | 114 |
| 19.24 | 1149 | 108 | 581 | 135 |
| 19.26 | 1043 | 128 | 543 | 117 |
| 19.28 | 773 | 130 | 576 | 114 |
| 19.3 | 562 | 142 | 543 | 110 |
| 19.32 | 488 | 128 | 557 | 117 |
| 19.34 | 458 | 121 | 590 | 106 |
| 19.36 | 380 | 142 | 600 | 132 |
| 19.38 | 380 | 166 | 615 | 128 |
| 19.4 | 384 | 174 | 566 | 144 |
| 19.42 | 408 | 151 | 686 | 130 |
| 19.44 | 424 | 169 | 713 | 146 |
| 19.46 | 441 | 199 | 778 | 154 |
| 19.48 | 441 | 188 | 773 | 164 |
| 19.5 | 506 | 180 | 745 | 154 |
| 19.52 | 484 | 180 | 801 | 174 |
| 19.54 | 534 | 185 | 773 | 144 |
| 19.56 | 671 | 202 | 762 | 149 |
| 19.58 | 625 | 196 | 740 | 161 |
| 19.6 | 552 | 213 | 692 | 169 |
| 19.62 | 686 | 234 | 713 | 210 |
| 19.64 | 751 | 196 | 660 | 185 |
| 19.66 | 762 | 210 | 630 | 199 |
| 19.68 | 864 | 219 | 620 | 199 |
| 19.7 | 1089 | 188 | 650 | 207 |
| 19.72 | 1163 | 202 | 666 | 213 |
| 19.74 | 1170 | 228 | 692 | 207 |
| 19.76 | 1414 | 253 | 620 | 262 |
| 19.78 | 1739 | 246 | 645 | 279 |
| 19.8 | 1632 | 248 | 625 | 253 |
| 19.82 | 1576 | 262 | 595 | 292 |
| 19.84 | 1475 | 272 | 625 | 306 |
| 19.86 | 1467 | 246 | 576 | 259 |
| 19.88 | 1498 | 225 | 534 | 276 |
| 19.9 | 1376 | 266 | 600 | 246 |
| 19.92 | 1096 | 256 | 543 | 207 |
| 19.94 | 692 | 292 | 645 | 190 |
| 19.96 | 524 | 259 | 650 | 219 |
| 19.98 | 441 | 372 | 660 | 240 |
| 20 | 292 | 400 | 610 | 246 |
| 20.02 | 266 | 404 | 718 | 262 |
| 20.04 | 256 | 471 | 708 | 253 |
| 20.06 | 313 | 515 | 734 | 331 |
| 20.08 | 339 | 562 | 767 | 380 |
| 20.1 | 306 | 571 | 790 | 392 |
| 20.12 | 320 | 671 | 847 | 420 |
| 20.14 | 328 | 702 | 858 | 441 |
| 20.16 | 350 | 829 | 829 | 515 |
| 20.18 | 353 | 853 | 888 | 467 |
| 20.2 | 331 | 930 | 829 | 576 |
| 20.22 | 372 | 900 | 751 | 543 |
| 20.24 | 369 | 1030 | 745 | 615 |
| 20.26 | 392 | 1018 | 676 | 681 |
| 20.28 | 424 | 1056 | 620 | 671 |
| 20.3 | 416 | 986 | 640 | 713 |
| 20.32 | 420 | 1005 | 586 | 702 |
| 20.34 | 408 | 818 | 610 | 692 |
| 20.36 | 416 | 686 | 640 | 615 |
| 20.38 | 384 | 488 | 655 | 511 |
| 20.4 | 372 | 353 | 600 | 471 |
| 20.42 | 380 | 296 | 713 | 339 |
| 20.44 | 388 | 222 | 645 | 279 |
| 20.46 | 357 | 190 | 630 | 231 |
| 20.48 | 376 | 154 | 620 | 180 |
| 20.5 | 372 | 161 | 600 | 164 |
| 20.52 | 365 | 156 | 600 | 154 |
| 20.54 | 384 | 172 | 552 | 144 |
| 20.56 | 484 | 182 | 586 | 166 |
| 20.58 | 433 | 210 | 493 | 151 |
| 20.6 | 441 | 185 | 538 | 156 |
| 20.62 | 471 | 199 | 529 | 193 |
| 20.64 | 458 | 231 | 488 | 185 |
| 20.66 | 548 | 246 | 488 | 190 |
| 20.68 | 650 | 289 | 543 | 202 |
| 20.7 | 697 | 276 | 475 | 210 |
| 20.72 | 812 | 306 | 475 | 246 |
| 20.74 | 980 | 353 | 506 | 279 |
| 20.76 | 1204 | 350 | 520 | 276 |
| 20.78 | 955 | 424 | 529 | 303 |
| 20.8 | 986 | 437 | 586 | 317 |
| 20.82 | 812 | 475 | 595 | 335 |
| 20.84 | 538 | 520 | 562 | 412 |
| 20.86 | 529 | 600 | 605 | 437 |
| 20.88 | 449 | 676 | 650 | 524 |
| 20.9 | 404 | 686 | 686 | 511 |
| 20.92 | 400 | 713 | 676 | 576 |
| 20.94 | 433 | 767 | 740 | 650 |
| 20.96 | 433 | 829 | 762 | 713 |
| 20.98 | 462 | 773 | 824 | 713 |
| 21 | 467 | 807 | 767 | 713 |
| 21.02 | 449 | 745 | 870 | 729 |
| 21.04 | 420 | 713 | 847 | 686 |
| 21.06 | 404 | 640 | 882 | 660 |
| 21.08 | 376 | 620 | 888 | 676 |
| 21.1 | 342 | 595 | 870 | 620 |
| 21.12 | 350 | 543 | 847 | 615 |
| 21.14 | 324 | 576 | 778 | 605 |
| 21.16 | 350 | 615 | 745 | 702 |
| 21.18 | 369 | 630 | 655 | 756 |
| 21.2 | 380 | 645 | 615 | 818 |
| 21.22 | 361 | 660 | 524 | 955 |
| 21.24 | 339 | 666 | 543 | 1011 |
| 21.26 | 296 | 581 | 515 | 1076 |
| 21.28 | 262 | 562 | 511 | 967 |
| 21.3 | 246 | 412 | 520 | 824 |
| 21.32 | 240 | 365 | 511 | 692 |
| 21.34 | 202 | 262 | 484 | 548 |
| 21.36 | 219 | 222 | 524 | 392 |
| 21.38 | 188 | 185 | 562 | 324 |
| 21.4 | 207 | 159 | 590 | 234 |
| 21.42 | 199 | 139 | 590 | 169 |
| 21.44 | 199 | 128 | 620 | 159 |
| 21.46 | 199 | 144 | 562 | 144 |
| 21.48 | 231 | 144 | 620 | 139 |
| 21.5 | 182 | 119 | 625 | 130 |
| 21.52 | 202 | 151 | 666 | 159 |
| 21.54 | 174 | 125 | 660 | 130 |
| 21.56 | 207 | 142 | 660 | 123 |
| 21.58 | 196 | 112 | 740 | 123 |
| 21.6 | 237 | 154 | 724 | 139 |
| 21.62 | 213 | 139 | 650 | 149 |
| 21.64 | 193 | 125 | 708 | 137 |
| 21.66 | 213 | 110 | 751 | 135 |
| 21.68 | 213 | 119 | 795 | 132 |
| 21.7 | 213 | 114 | 756 | 128 |
| 21.72 | 272 | 137 | 734 | 159 |
| 21.74 | 276 | 174 | 734 | 132 |
| 21.76 | 286 | 151 | 702 | 142 |
| 21.78 | 286 | 169 | 686 | 166 |
| 21.8 | 306 | 174 | 640 | 144 |
| 21.82 | 289 | 182 | 620 | 174 |
| 21.84 | 306 | 213 | 605 | 219 |
| 21.86 | 289 | 225 | 557 | 216 |
| 21.88 | 324 | 306 | 576 | 234 |
| 21.9 | 342 | 276 | 590 | 276 |
| 21.92 | 412 | 299 | 640 | 286 |
| 21.94 | 433 | 331 | 615 | 282 |
| 21.96 | 462 | 350 | 686 | 353 |
| 21.98 | 515 | 376 | 660 | 433 |
| 22 | 515 | 433 | 702 | 454 |
| 22.02 | 586 | 480 | 640 | 445 |
| 22.04 | 708 | 625 | 697 | 529 |
| 22.06 | 686 | 562 | 762 | 534 |
| 22.08 | 992 | 625 | 734 | 586 |
| 22.1 | 1170 | 630 | 713 | 671 |
| 22.12 | 1459 | 697 | 724 | 778 |
| 22.14 | 1303 | 767 | 708 | 870 |
| 22.16 | 999 | 795 | 692 | 864 |
| 22.18 | 812 | 858 | 708 | 912 |
| 22.2 | 645 | 900 | 671 | 1024 |
| 22.22 | 458 | 955 | 666 | 1050 |
| 22.24 | 313 | 900 | 734 | 1037 |
| 22.26 | 306 | 835 | 773 | 942 |
| 22.28 | 269 | 660 | 778 | 894 |
| 22.3 | 246 | 595 | 773 | 801 |
| 22.32 | 243 | 484 | 807 | 635 |
| 22.34 | 240 | 454 | 835 | 524 |
| 22.36 | 250 | 437 | 795 | 428 |
| 22.38 | 246 | 420 | 841 | 400 |
| 22.4 | 243 | 404 | 894 | 384 |
| 22.42 | 276 | 441 | 824 | 365 |
| 22.44 | 222 | 346 | 894 | 380 |
| 22.46 | 246 | 335 | 942 | 342 |
| 22.48 | 234 | 361 | 942 | 331 |
| 22.5 | 202 | 237 | 1076 | 350 |
| 22.52 | 222 | 210 | 1050 | 259 |
| 22.54 | 216 | 180 | 1018 | 250 |
| 22.56 | 210 | 161 | 1096 | 210 |
| 22.58 | 199 | 151 | 1018 | 185 |
| 22.6 | 182 | 121 | 986 | 196 |
| 22.62 | 151 | 112 | 980 | 159 |
| 22.64 | 164 | 125 | 882 | 161 |
| 22.66 | 177 | 110 | 807 | 166 |
| 22.68 | 193 | 144 | 734 | 159 |
| 22.7 | 196 | 146 | 630 | 159 |
| 22.72 | 188 | 123 | 557 | 174 |
| 22.74 | 188 | 125 | 605 | 146 |
| 22.76 | 246 | 121 | 515 | 172 |
| 22.78 | 193 | 159 | 497 | 154 |
| 22.8 | 256 | 174 | 493 | 149 |
| 22.82 | 243 | 146 | 529 | 190 |
| 22.84 | 246 | 164 | 484 | 174 |
| 22.86 | 250 | 174 | 488 | 182 |
| 22.88 | 262 | 188 | 467 | 193 |
| 22.9 | 279 | 164 | 454 | 190 |
| 22.92 | 282 | 185 | 454 | 253 |
| 22.94 | 299 | 210 | 445 | 237 |
| 22.96 | 303 | 204 | 437 | 256 |
| 22.98 | 317 | 199 | 412 | 276 |
| 23 | 306 | 216 | 424 | 310 |
| 23.02 | 384 | 269 | 467 | 303 |
| 23.04 | 365 | 246 | 416 | 331 |
| 23.06 | 342 | 289 | 471 | 388 |
| 23.08 | 361 | 282 | 445 | 428 |
| 23.1 | 292 | 313 | 458 | 502 |
| 23.12 | 313 | 384 | 484 | 543 |
| 23.14 | 243 | 365 | 538 | 520 |
| 23.16 | 210 | 376 | 562 | 562 |
| 23.18 | 180 | 384 | 524 | 615 |
| 23.2 | 196 | 342 | 557 | 595 |
| 23.22 | 240 | 306 | 586 | 600 |
| 23.24 | 199 | 346 | 630 | 620 |
| 23.26 | 199 | 317 | 605 | 630 |
| 23.28 | 202 | 296 | 635 | 605 |
| 23.3 | 216 | 289 | 681 | 600 |
| 23.32 | 240 | 286 | 635 | 640 |
| 23.34 | 210 | 339 | 645 | 676 |
| 23.36 | 207 | 350 | 666 | 734 |
| 23.38 | 225 | 396 | 708 | 824 |
| 23.4 | 222 | 388 | 697 | 847 |
| 23.42 | 237 | 416 | 650 | 961 |
| 23.44 | 243 | 543 | 635 | 1082 |
| 23.46 | 240 | 571 | 635 | 1267 |
| 23.48 | 286 | 745 | 620 | 1391 |
| 23.5 | 292 | 1109 | 615 | 1490 |
| 23.52 | 310 | 1354 | 552 | 1640 |
| 23.54 | 286 | 1069 | 529 | 1849 |
| 23.56 | 292 | 762 | 467 | 2209 |
| 23.58 | 313 | 681 | 445 | 2052 |
| 23.6 | 282 | 475 | 437 | 1225 |
| 23.62 | 286 | 299 | 428 | 1122 |
| 23.64 | 328 | 243 | 420 | 900 |
| 23.66 | 346 | 234 | 424 | 475 |
| 23.68 | 361 | 216 | 480 | 328 |
| 23.7 | 350 | 213 | 441 | 253 |
| 23.72 | 342 | 219 | 458 | 243 |
| 23.74 | 372 | 210 | 475 | 256 |
| 23.76 | 400 | 222 | 506 | 250 |
| 23.78 | 408 | 210 | 506 | 350 |
| 23.8 | 384 | 196 | 484 | 222 |
| 23.82 | 357 | 164 | 506 | 188 |
| 23.84 | 306 | 164 | 484 | 204 |
| 23.86 | 253 | 166 | 480 | 196 |
| 23.88 | 246 | 139 | 462 | 161 |
| 23.9 | 240 | 117 | 433 | 151 |
| 23.92 | 174 | 96 | 376 | 123 |
| 23.94 | 154 | 106 | 372 | 132 |
| 23.96 | 132 | 110 | 388 | 132 |
| 23.98 | 174 | 92 | 357 | 119 |
| 24 | 142 | 96 | 392 | 125 |
| 24.02 | 139 | 104 | 350 | 154 |
| 24.04 | 144 | 90 | 353 | 100 |
| 24.06 | 166 | 112 | 380 | 128 |
| 24.08 | 123 | 85 | 357 | 135 |
| 24.1 | 137 | 98 | 328 | 114 |
| 24.12 | 149 | 123 | 369 | 128 |
| 24.14 | 139 | 125 | 357 | 119 |
| 24.16 | 159 | 112 | 384 | 149 |
| 24.18 | 130 | 135 | 400 | 144 |
| 24.2 | 164 | 135 | 372 | 159 |
| 24.22 | 164 | 144 | 404 | 164 |
| 24.24 | 177 | 177 | 372 | 164 |
| 24.26 | 172 | 185 | 369 | 193 |
| 24.28 | 207 | 161 | 388 | 188 |
| 24.3 | 164 | 222 | 408 | 234 |
| 24.32 | 182 | 216 | 365 | 207 |
| 24.34 | 188 | 234 | 392 | 253 |
| 24.36 | 196 | 225 | 467 | 276 |
| 24.38 | 216 | 256 | 388 | 361 |
| 24.4 | 213 | 262 | 475 | 335 |
| 24.42 | 207 | 286 | 428 | 342 |
| 24.44 | 222 | 286 | 445 | 369 |
| 24.46 | 253 | 313 | 515 | 396 |
| 24.48 | 216 | 342 | 506 | 396 |
| 24.5 | 231 | 400 | 475 | 471 |
| 24.52 | 253 | 380 | 493 | 467 |
| 24.54 | 286 | 424 | 520 | 538 |
| 24.56 | 237 | 428 | 511 | 515 |
| 24.58 | 256 | 467 | 524 | 497 |
| 24.6 | 286 | 462 | 506 | 529 |
| 24.62 | 339 | 357 | 552 | 576 |
| 24.64 | 299 | 388 | 538 | 552 |
| 24.66 | 259 | 286 | 511 | 462 |
| 24.68 | 234 | 289 | 538 | 433 |
| 24.7 | 193 | 246 | 581 | 335 |
| 24.72 | 172 | 213 | 562 | 269 |
| 24.74 | 172 | 196 | 557 | 199 |
| 24.76 | 169 | 196 | 625 | 225 |
| 24.78 | 172 | 193 | 655 | 202 |
| 24.8 | 161 | 216 | 645 | 193 |
| 24.82 | 159 | 199 | 605 | 188 |
| 24.84 | 166 | 199 | 666 | 190 |
| 24.86 | 174 | 225 | 650 | 210 |
| 24.88 | 169 | 222 | 615 | 228 |
| 24.9 | 161 | 272 | 581 | 250 |
| 24.92 | 159 | 259 | 552 | 231 |
| 24.94 | 159 | 213 | 506 | 222 |
| 24.96 | 159 | 256 | 462 | 188 |
| 24.98 | 156 | 213 | 462 | 213 |
| 25 | 174 | 180 | 454 | 199 |
| 25.02 | 169 | 172 | 420 | 202 |
| 25.04 | 193 | 161 | 416 | 172 |
| 25.06 | 207 | 164 | 404 | 159 |
| 25.08 | 193 | 135 | 467 | 149 |
| 25.1 | 213 | 144 | 458 | 151 |
| 25.12 | 234 | 130 | 441 | 146 |
| 25.14 | 202 | 121 | 480 | 121 |
| 25.16 | 199 | 125 | 445 | 110 |
| 25.18 | 202 | 149 | 480 | 117 |
| 25.2 | 193 | 112 | 458 | 121 |
| 25.22 | 228 | 106 | 467 | 130 |
| 25.24 | 185 | 112 | 493 | 339 |
| 25.26 | 202 | 112 | 471 | 108 |
| 25.28 | 225 | 128 | 484 | 121 |
| 25.3 | 250 | 161 | 502 | 137 |
| 25.32 | 269 | 123 | 524 | 128 |
| 25.34 | 259 | 128 | 524 | 121 |
| 25.36 | 237 | 154 | 493 | 137 |
| 25.38 | 262 | 142 | 529 | 161 |
| 25.4 | 276 | 146 | 581 | 172 |
| 25.42 | 219 | 142 | 571 | 149 |
| 25.44 | 207 | 164 | 571 | 169 |
| 25.46 | 188 | 169 | 557 | 180 |
| 25.48 | 207 | 166 | 590 | 159 |
| 25.5 | 188 | 266 | 529 | 159 |
| 25.52 | 190 | 169 | 471 | 144 |
| 25.54 | 193 | 164 | 475 | 128 |
| 25.56 | 182 | 166 | 449 | 154 |
| 25.58 | 207 | 204 | 445 | 128 |
| 25.6 | 161 | 210 | 420 | 180 |
| 25.62 | 169 | 269 | 400 | 149 |
| 25.64 | 185 | 228 | 412 | 172 |
| 25.66 | 185 | 266 | 372 | 177 |
| 25.68 | 174 | 266 | 404 | 182 |
| 25.7 | 193 | 292 | 328 | 182 |
| 25.72 | 185 | 269 | 346 | 219 |
| 25.74 | 161 | 286 | 342 | 182 |
| 25.76 | 154 | 289 | 346 | 210 |
| 25.78 | 166 | 365 | 331 | 219 |
| 25.8 | 196 | 296 | 331 | 222 |
| 25.82 | 190 | 266 | 331 | 246 |
| 25.84 | 169 | 279 | 342 | 246 |
| 25.86 | 188 | 276 | 306 | 243 |
| 25.88 | 166 | 320 | 313 | 219 |
| 25.9 | 185 | 296 | 328 | 222 |
| 25.92 | 484 | 324 | 279 | 237 |
| 25.94 | 169 | 310 | 279 | 237 |
| 25.96 | 177 | 299 | 299 | 213 |
| 25.98 | 188 | 292 | 310 | 199 |
| 26 | 159 | 259 | 310 | 204 |
| 26.02 | 161 | 292 | 292 | 207 |
| 26.04 | 177 | 222 | 313 | 202 |
| 26.06 | 154 | 207 | 296 | 177 |
| 26.08 | 149 | 156 | 331 | 164 |
| 26.1 | 189 | 158 | 289 | 135 |
| 26.12 | 144 | 125 | 320 | 130 |
| 26.14 | 139 | 142 | 292 | 132 |
| 26.16 | 114 | 108 | 303 | 146 |
| 26.18 | 144 | 104 | 292 | 114 |
| 26.2 | 135 | 112 | 317 | 110 |
| 26.22 | 144 | 104 | 320 | 123 |
| 26.24 | 142 | 106 | 324 | 117 |
| 26.26 | 164 | 128 | 292 | 144 |
| 26.28 | 164 | 130 | 313 | 132 |
| 26.3 | 177 | 130 | 299 | 125 |
| 26.32 | 180 | 137 | 328 | 142 |
| 26.34 | 196 | 142 | 328 | 135 |
| 26.36 | 225 | 185 | 320 | 202 |
| 26.38 | 279 | 177 | 342 | 161 |
| 26.4 | 310 | 207 | 350 | 188 |
| 26.42 | 306 | 207 | 331 | 159 |
| 26.44 | 342 | 228 | 369 | 164 |
| 26.46 | 335 | 199 | 365 | 189 |
| 26.48 | 353 | 225 | 353 | 174 |
| 26.5 | 331 | 237 | 388 | 166 |
| 26.52 | 369 | 240 | 416 | 185 |
| 26.54 | 372 | 272 | 424 | 222 |
| 26.56 | 454 | 262 | 437 | 196 |
| 26.58 | 433 | 313 | 384 | 202 |
| 26.6 | 529 | 317 | 449 | 243 |
| 26.62 | 655 | 335 | 458 | 250 |
| 26.64 | 858 | 350 | 458 | 246 |
| 26.66 | 1109 | 320 | 454 | 256 |
| 26.68 | 1490 | 380 | 497 | 253 |
| 26.7 | 1347 | 331 | 454 | 234 |
| 26.72 | 1102 | 317 | 449 | 282 |
| 26.74 | 1063 | 335 | 458 | 269 |
| 26.76 | 1030 | 324 | 408 | 286 |
| 26.78 | 894 | 328 | 412 | 272 |
| 26.8 | 610 | 320 | 376 | 269 |
| 26.82 | 497 | 365 | 376 | 292 |
| 26.84 | 408 | 342 | 420 | 339 |
| 26.86 | 396 | 376 | 388 | 342 |
| 26.88 | 350 | 365 | 376 | 286 |
| 26.9 | 292 | 372 | 416 | 320 |
| 26.92 | 328 | 339 | 388 | 313 |
| 26.94 | 331 | 342 | 445 | 353 |
| 26.96 | 346 | 303 | 412 | 328 |
| 26.98 | 384 | 289 | 441 | 299 |
| 27 | 412 | 279 | 380 | 331 |
| 27.02 | 384 | 246 | 416 | 266 |
| 27.04 | 412 | 213 | 433 | 289 |
| 27.06 | 441 | 231 | 353 | 262 |
| 27.08 | 471 | 196 | 404 | 272 |
| 27.1 | 458 | 213 | 396 | 279 |
| 27.12 | 416 | 190 | 372 | 228 |
| 27.14 | 475 | 190 | 388 | 240 |
| 27.16 | 511 | 169 | 380 | 246 |
| 27.18 | 493 | 159 | 353 | 250 |
| 27.2 | 480 | 139 | 412 | 222 |
| 27.22 | 534 | 169 | 342 | 185 |
| 27.24 | 543 | 154 | 400 | 169 |
| 27.26 | 458 | 174 | 454 | 169 |
| 27.28 | 437 | 169 | 441 | 172 |
| 27.3 | 462 | 159 | 467 | 193 |
| 27.32 | 420 | 154 | 433 | 188 |
| 27.34 | 313 | 174 | 445 | 161 |
| 27.36 | 243 | 182 | 428 | 166 |
| 27.38 | 246 | 182 | 441 | 174 |
| 27.4 | 222 | 174 | 400 | 188 |
| 27.42 | 199 | 177 | 471 | 190 |
| 27.44 | 166 | 177 | 458 | 174 |
| 27.46 | 156 | 154 | 441 | 207 |
| 27.48 | 144 | 172 | 475 | 164 |
| 27.5 | 166 | 174 | 458 | 169 |
| 27.52 | 166 | 144 | 428 | 137 |
| 27.54 | 159 | 174 | 433 | 123 |
| 27.56 | 159 | 139 | 400 | 149 |
| 27.58 | 164 | 172 | 420 | 137 |
| 27.6 | 161 | 156 | 384 | 151 |
| 27.62 | 164 | 149 | 361 | 123 |
| 27.64 | 161 | 123 | 361 | 135 |
| 27.66 | 188 | 159 | 384 | 128 |
| 27.68 | 204 | 142 | 396 | 154 |
| 27.7 | 193 | 144 | 392 | 169 |
| 27.72 | 219 | 135 | 404 | 156 |
| 27.74 | 204 | 128 | 420 | 164 |
| 27.76 | 199 | 156 | 441 | 185 |
| 27.78 | 216 | 154 | 412 | 180 |
| 27.8 | 231 | 159 | 441 | 231 |
| 27.82 | 234 | 156 | 428 | 190 |
| 27.84 | 313 | 193 | 506 | 204 |
| 27.86 | 306 | 172 | 502 | 210 |
| 27.88 | 306 | 172 | 515 | 231 |
| 27.9 | 335 | 185 | 511 | 210 |
| 27.92 | 313 | 204 | 511 | 231 |
| 27.94 | 317 | 237 | 562 | 253 |
| 27.96 | 317 | 237 | 548 | 250 |
| 27.98 | 324 | 243 | 600 | 276 |
| 28 | 303 | 256 | 557 | 320 |
| 28.02 | 306 | 256 | 557 | 303 |
| 28.04 | 269 | 286 | 566 | 282 |
| 28.06 | 243 | 279 | 543 | 342 |
| 28.08 | 231 | 286 | 576 | 372 |
| 28.1 | 219 | 299 | 548 | 369 |
| 28.12 | 210 | 306 | 576 | 342 |
| 28.14 | 219 | 250 | 576 | 388 |
| 28.16 | 202 | 234 | 557 | 361 |
| 28.18 | 199 | 204 | 586 | 306 |
| 28.2 | 225 | 196 | 520 | 243 |
| 28.22 | 161 | 182 | 529 | 202 |
| 28.24 | 177 | 159 | 497 | 188 |
| 28.26 | 169 | 151 | 480 | 188 |
| 28.28 | 210 | 144 | 420 | 177 |
| 28.3 | 222 | 132 | 467 | 161 |
| 28.32 | 202 | 146 | 449 | 137 |
| 28.34 | 237 | 142 | 484 | 142 |
| 28.36 | 256 | 125 | 428 | 142 |
| 28.38 | 234 | 156 | 424 | 114 |
| 28.4 | 234 | 146 | 437 | 130 |
| 28.42 | 286 | 159 | 437 | 142 |
| 28.44 | 306 | 161 | 420 | 174 |
| 28.46 | 339 | 177 | 4.45 | 135 |
| 28.48 | 310 | 172 | 458 | 166 |
| 28.5 | 303 | 169 | 416 | 169 |
| 28.52 | 276 | 174 | 437 | 159 |
| 28.54 | 259 | 182 | 458 | 156 |
| 28.56 | 256 | 169 | 437 | 149 |
| 28.58 | 240 | 169 | 449 | 174 |
| 28.6 | 216 | 172 | 433 | 161 |
| 28.62 | 231 | 174 | 441 | 149 |
| 28.64 | 237 | 174 | 445 | 185 |
| 28.66 | 234 | 177 | 433 | 161 |
| 28.68 | 259 | 207 | 424 | 144 |
| 28.7 | 213 | 185 | 416 | 169 |
| 28.72 | 180 | 185 | 408 | 144 |
| 28.74 | 169 | 174 | 441 | 139 |
| 28.76 | 159 | 164 | 420 | 144 |
| 28.78 | 159 | 166 | 408 | 144 |
| 28.8 | 144 | 156 | 458 | 151 |
| 28.82 | 174 | 137 | 388 | 117 |
| 28.84 | 149 | 128 | 408 | 125 |
| 28.86 | 144 | 130 | 416 | 117 |
| 28.88 | 149 | 100 | 420 | 119 |
| 28.9 | 151 | 106 | 380 | 117 |
| 28.92 | 156 | 110 | 428 | 108 |
| 28.94 | 132 | 88 | 404 | 144 |
| 28.96 | 159 | 98 | 376 | 123 |
| 28.98 | 199 | 88 | 396 | 123 |
| 29 | 169 | 119 | 365 | 110 |
| 29.02 | 199 | 104 | 342 | 123 |
| 29.04 | 193 | 144 | 342 | 151 |
| 29.06 | 228 | 117 | 335 | 159 |
| 29.08 | 210 | 130 | 357 | 169 |
| 29.1 | 216 | 132 | 296 | 164 |
| 29.12 | 246 | 137 | 317 | 151 |
| 29.14 | 246 | 151 | 361 | 174 |
| 29.16 | 276 | 159 | 303 | 185 |
| 29.18 | 253 | 182 | 289 | 225 |
| 29.2 | 269 | 182 | 310 | 199 |
| 29.22 | 246 | 172 | 339 | 219 |
| 29.24 | 266 | 196 | 317 | 231 |
| 29.26 | 228 | 207 | 339 | 234 |
| 29.28 | 240 | 202 | 328 | 234 |
| 29.3 | 234 | 225 | 324 | 266 |
| 29.32 | 210 | 228 | 313 | 282 |
| 29.34 | 202 | 243 | 306 | 320 |
| 29.36 | 177 | 266 | 335 | 339 |
| 29.38 | 204 | 286 | 299 | 342 |
| 29.4 | 169 | 320 | 335 | 388 |
| 29.42 | 177 | 292 | 282 | 388 |
| 29.44 | 159 | 335 | 320 | 384 |
| 29.46 | 182 | 313 | 328 | 416 |
| 29.48 | 169 | 339 | 324 | 437 |
| 29.5 | 169 | 353 | 306 | 416 |
| 29.52 | 159 | 306 | 342 | 335 |
| 29.54 | 149 | 306 | 376 | 365 |
| 29.56 | 149 | 279 | 350 | 328 |
| 29.58 | 156 | 324 | 388 | 303 |
| 29.6 | 159 | 303 | 376 | 279 |
| 29.62 | 156 | 269 | 369 | 282 |
| 29.64 | 161 | 276 | 372 | 259 |
| 29.66 | 174 | 303 | 372 | 266 |
| 29.68 | 207 | 250 | 420 | 234 |
| 29.7 | 199 | 320 | 388 | 262 |
| 29.72 | 210 | 276 | 396 | 259 |
| 29.74 | 210 | 299 | 400 | 240 |
| 29.76 | 190 | 328 | 384 | 269 |
| 29.78 | 180 | 269 | 420 | 282 |
| 29.8 | 199 | 339 | 449 | 262 |
| 29.82 | 210 | 269 | 416 | 276 |
| 29.84 | 219 | 276 | 400 | 262 |
| 29.86 | 207 | 276 | 404 | 253 |
| 29.88 | 225 | 228 | 400 | 259 |
| 29.9 | 185 | 234 | 412 | 250 |
| 29.92 | 210 | 213 | 408 | 202 |
| 29.94 | 213 | 216 | 384 | 204 |
| 29.96 | 231 | 196 | 396 | 210 |
| 29.98 | 222 | 177 | 384 | 193 |
| 30 | 234 | 166 | 365 | 166 |
| 30.02 | 213 | 139 | 372 | 182 |
| 30.04 | 231 | 137 | 376 | 159 |
| 30.06 | 259 | 112 | 384 | 128 |
| 30.08 | 222 | 102 | 376 | 146 |
| 30.1 | 182 | 108 | 357 | 128 |
| 30.12 | 166 | 90 | 384 | 114 |
| 30.14 | 185 | 98 | 357 | 104 |
| 30.16 | 169 | 100 | 365 | 121 |
| 30.18 | 123 | 123 | 353 | 110 |
| 30.2 | 164 | 114 | 346 | 117 |
| 30.22 | 159 | 106 | 320 | 112 |
| 30.24 | 149 | 88 | 357 | 128 |
| 30.26 | 146 | 114 | 339 | 123 |
| 30.28 | 225 | 123 | 365 | 128 |
| 30.3 | 144 | 130 | 339 | 112 |
| 30.32 | 164 | 117 | 357 | 125 |
| 30.34 | 132 | 137 | 365 | 125 |
| 30.36 | 144 | 144 | 376 | 108 |
| 30.38 | 149 | 123 | 350 | 144 |
| 30.4 | 207 | 137 | 328 | 123 |
| 30.42 | 159 | 151 | 365 | 128 |
| 30.44 | 234 | 142 | 361 | 144 |
| 30.46 | 174 | 151 | 376 | 159 |
| 30.48 | 185 | 169 | 380 | 146 |
| 30.5 | 207 | 166 | 380 | 166 |
| 30.52 | 193 | 177 | 342 | 151 |
| 30.54 | 219 | 156 | 376 | 169 |
| 30.56 | 243 | 193 | 365 | 164 |
| 30.58 | 219 | 169 | 353 | 139 |
| 30.6 | 253 | 169 | 404 | 177 |
| 30.62 | 246 | 166 | 335 | 146 |
| 30.64 | 303 | 169 | 408 | 144 |
| 30.66 | 357 | 146 | 365 | 159 |
| 30.68 | 408 | 177 | 365 | 149 |
| 30.7 | 416 | 166 | 376 | 180 |
| 30.72 | 365 | 137 | 353 | 164 |
| 30.74 | 400 | 193 | 372 | 190 |
| 30.76 | 380 | 174 | 404 | 174 |
| 30.78 | 365 | 177 | 437 | 180 |
| 30.8 | 365 | 185 | 412 | 177 |
| 30.82 | 306 | 174 | 412 | 199 |
| 30.84 | 272 | 177 | 380 | 177 |
| 30.86 | 262 | 161 | 416 | 204 |
| 30.88 | 250 | 151 | 471 | 161 |
| 30.9 | 228 | 149 | 445 | 164 |
| 30.92 | 202 | 172 | 462 | 146 |
| 30.94 | 185 | 161 | 458 | 149 |
| 30.96 | 164 | 169 | 437 | 177 |
| 30.98 | 269 | 185 | 441 | 174 |
| 31 | 182 | 180 | 428 | 144 |
| 31.02 | 193 | 172 | 445 | 169 |
| 31.04 | 207 | 193 | 449 | 185 |
| 31.06 | 216 | 190 | 441 | 166 |
| 31.08 | 234 | 182 | 420 | 164 |
| 31.1 | 228 | 161 | 437 | 154 |
| 31.12 | 190 | 190 | 416 | 154 |
| 31.14 | 202 | 182 | 449 | 169 |
| 31.16 | 282 | 121 | 424 | 159 |
| 31.18 | 279 | 128 | 445 | 144 |
| 31.2 | 276 | 119 | 400 | 137 |
| 31.22 | 237 | 100 | 408 | 142 |
| 31.24 | 234 | 121 | 380 | 121 |
| 31.26 | 231 | 108 | 313 | 123 |
| 31.28 | 250 | 110 | 365 | 132 |
| 31.3 | 193 | 104 | 339 | 104 |
| 31.32 | 240 | 106 | 342 | 119 |
| 31.34 | 222 | 108 | 313 | 110 |
| 31.36 | 196 | 106 | 350 | 114 |
| 31.38 | 190 | 121 | 350 | 125 |
| 31.4 | 204 | 144 | 320 | 119 |
| 31.42 | 161 | 128 | 335 | 164 |
| 31.44 | 202 | 144 | 335 | 135 |
| 31.46 | 188 | 144 | 266 | 159 |
| 31.48 | 177 | 177 | 306 | 216 |
| 31.5 | 159 | 193 | 286 | 207 |
| 31.52 | 185 | 207 | 324 | 210 |
| 31.54 | 166 | 219 | 313 | 225 |
| 31.56 | 166 | 228 | 313 | 259 |
| 31.58 | 174 | 259 | 317 | 269 |
| 31.6 | 177 | 279 | 313 | 292 |
| 31.62 | 182 | 292 | 324 | 303 |
| 31.64 | 213 | 320 | 339 | 310 |
| 31.66 | 207 | 324 | 335 | 310 |
| 31.68 | 213 | 365 | 331 | 331 |
| 31.7 | 207 | 369 | 335 | 404 |
| 31.72 | 240 | 365 | 357 | 416 |
| 31.74 | 228 | 396 | 339 | 502 |
| 31.76 | 243 | 420 | 357 | 497 |
| 31.78 | 237 | 433 | 328 | 502 |
| 31.8 | 231 | 475 | 350 | 534 |
| 31.82 | 231 | 511 | 369 | 502 |
| 31.84 | 210 | 493 | 384 | 520 |
| 31.86 | 199 | 484 | 372 | 605 |
| 31.88 | 225 | 484 | 350 | 586 |
| 31.9 | 210 | 433 | 339 | 590 |
| 31.92 | 210 | 400 | 346 | 506 |
| 31.94 | 228 | 369 | 342 | 497 |
| 31.96 | 199 | 313 | 331 | 441 |
| 31.98 | 210 | 310 | 350 | 384 |
| 32 | 199 | 253 | 306 | 328 |
| 32.02 | 190 | 196 | 317 | 296 |
| 32.04 | 177 | 216 | 317 | 246 |
| 32.06 | 193 | 182 | 313 | 259 |
| 32.08 | 174 | 180 | 331 | 199 |
| 32.1 | 166 | 185 | 317 | 225 |
| 32.12 | 156 | 190 | 328 | 188 |
| 32.14 | 182 | 190 | 331 | 216 |
| 32.16 | 144 | 207 | 328 | 213 |
| 32.18 | 151 | 207 | 353 | 240 |
| 32.2 | 132 | 188 | 342 | 228 |
| 32.22 | 142 | 216 | 372 | 256 |
| 32.24 | 161 | 225 | 339 | 219 |
| 32.26 | 144 | 259 | 350 | 256 |
| 32.28 | 149 | 266 | 331 | 246 |
| 32.3 | 159 | 240 | 346 | 262 |
| 32.32 | 149 | 256 | 342 | 299 |
| 32.34 | 156 | 253 | 353 | 286 |
| 32.36 | 135 | 246 | 324 | 320 |
| 32.38 | 128 | 243 | 369 | 306 |
| 32.4 | 135 | 250 | 357 | 306 |
| 32.42 | 151 | 204 | 339 | 320 |
| 32.44 | 154 | 188 | 331 | 289 |
| 32.46 | 123 | 188 | 350 | 276 |
| 32.48 | 149 | 234 | 331 | 250 |
| 32.5 | 149 | 204 | 346 | 266 |
| 32.52 | 151 | 231 | 335 | 256 |
| 32.54 | 112 | 204 | 376 | 269 |
| 32.56 | 154 | 199 | 320 | 231 |
| 32.58 | 146 | 174 | 342 | 222 |
| 32.6 | 139 | 169 | 342 | 210 |
| 32.62 | 130 | 154 | 317 | 185 |
| 32.64 | 125 | 144 | 339 | 177 |
| 32.66 | 137 | 174 | 286 | 177 |
| 32.68 | 139 | 169 | 324 | 174 |
| 32.7 | 166 | 196 | 310 | 169 |
| 32.72 | 164 | 204 | 320 | 164 |
| 32.74 | 139 | 154 | 306 | 166 |
| 32.76 | 182 | 174 | 324 | 159 |
| 32.78 | 166 | 182 | 286 | 166 |
| 32.8 | 185 | 216 | 324 | 164 |
| 32.82 | 188 | 210 | 335 | 193 |
| 32.84 | 210 | 222 | 317 | 202 |
| 32.86 | 213 | 228 | 306 | 210 |
| 32.88 | 202 | 256 | 289 | 234 |
| 32.9 | 196 | 282 | 303 | 234 |
| 32.92 | 216 | 289 | 324 | 266 |
| 32.94 | 213 | 289 | 306 | 246 |
| 32.96 | 210 | 306 | 339 | 269 |
| 32.98 | 228 | 313 | 313 | 276 |
| 33 | 202 | 328 | 306 | 286 |
| 33.02 | 180 | 369 | 324 | 306 |
| 33.04 | 188 | 353 | 296 | 328 |
| 33.06 | 190 | 369 | 292 | 335 |
| 33.08 | 210 | 433 | 310 | 369 |
| 33.1 | 222 | 437 | 299 | 433 |
| 33.12 | 207 | 445 | 286 | 424 |
| 33.14 | 216 | 392 | 289 | 428 |
| 33.16 | 207 | 441 | 310 | 404 |
| 33.18 | 216 | 449 | 313 | 449 |
| 33.2 | 225 | 416 | 299 | 420 |
| 33.22 | 228 | 437 | 296 | 428 |
| 33.24 | 219 | 441 | 272 | 392 |
| 33.26 | 225 | 388 | 292 | 392 |
| 33.28 | 190 | 357 | 303 | 380 |
| 33.3 | 231 | 324 | 317 | 376 |
| 33.32 | 246 | 276 | 335 | 361 |
| 33.34 | 222 | 269 | 331 | 299 |
| 33.36 | 210 | 272 | 335 | 256 |
| 33.38 | 259 | 262 | 339 | 289 |
| 33.4 | 259 | 243 | 335 | 289 |
| 33.42 | 310 | 231 | 339 | 320 |
| 33.44 | 350 | 213 | 376 | 299 |
| 33.46 | 324 | 256 | 376 | 313 |
| 33.48 | 240 | 240 | 372 | 266 |
| 33.5 | 289 | 225 | 400 | 286 |
| 33.52 | 299 | 207 | 365 | 240 |
| 33.54 | 289 | 213 | 400 | 256 |
| 33.56 | 292 | 213 | 392 | 213 |
| 33.58 | 272 | 210 | 404 | 234 |
| 33.6 | 234 | 253 | 412 | 225 |
| 33.62 | 286 | 253 | 424 | 253 |
| 33.64 | 292 | 228 | 449 | 216 |
| 33.66 | 292 | 237 | 400 | 269 |
| 33.68 | 266 | 240 | 441 | 231 |
| 33.7 | 292 | 253 | 420 | 243 |
| 33.72 | 259 | 250 | 449 | 222 |
| 33.74 | 262 | 256 | 480 | 234 |
| 33.76 | 225 | 282 | 462 | 266 |
| 33.78 | 228 | 253 | 506 | 246 |
| 33.8 | 219 | 269 | 471 | 269 |
| 33.82 | 210 | 279 | 471 | 253 |
| 33.84 | 204 | 269 | 493 | 246 |
| 33.86 | 204 | 269 | 488 | 262 |
| 33.88 | 174 | 282 | 484 | 228 |
| 33.9 | 210 | 328 | 449 | 269 |
| 33.92 | 210 | 303 | 471 | 296 |
| 33.94 | 193 | 365 | 441 | 240 |
| 33.96 | 213 | 353 | 396 | 262 |
| 33.98 | 225 | 369 | 400 | 282 |
| 34 | 240 | 353 | 392 | 292 |
| 34.02 | 222 | 396 | 412 | 365 |
| 34.04 | 246 | 433 | 384 | 339 |
| 34.06 | 259 | 441 | 392 | 350 |
| 34.08 | 303 | 458 | 353 | 412 |
| 34.1 | 272 | 445 | 331 | 408 |
| 34.12 | 286 | 449 | 342 | 372 |
| 34.14 | 276 | 467 | 350 | 408 |
| 34.16 | 272 | 493 | 320 | 428 |
| 34.18 | 279 | 488 | 339 | 428 |
| 34.2 | 282 | 488 | 317 | 433 |
| 34.22 | 266 | 462 | 306 | 449 |
| 34.24 | 272 | 420 | 342 | 454 |
| 34.26 | 272 | 433 | 350 | 428 |
| 34.28 | 272 | 412 | 306 | 388 |
| 34.3 | 240 | 376 | 320 | 388 |
| 34.32 | 262 | 317 | 306 | 369 |
| 34.34 | 246 | 292 | 342 | 331 |
| 34.36 | 246 | 262 | 296 | 296 |
| 34.38 | 266 | 237 | 353 | 262 |
| 34.4 | 240 | 216 | 292 | 237 |
| 34.42 | 296 | 196 | 317 | 234 |
| 34.44 | 199 | 199 | 331 | 216 |
| 34.46 | 190 | 185 | 353 | 196 |
| 34.48 | 202 | 202 | 339 | 185 |
| 34.5 | 204 | 182 | 339 | 188 |
| 34.52 | 210 | 177 | 350 | 185 |
| 34.54 | 222 | 185 | 353 | 196 |
| 34.56 | 219 | 190 | 384 | 219 |
| 34.58 | 240 | 188 | 384 | 199 |
| 34.6 | 253 | 199 | 365 | 237 |
| 34.62 | 246 | 196 | 380 | 180 |
| 34.64 | 237 | 193 | 416 | 216 |
| 34.66 | 292 | 202 | 388 | 228 |
| 34.68 | 313 | 174 | 433 | 222 |
| 34.7 | 331 | 231 | 380 | 246 |
| 34.72 | 335 | 213 | 420 | 256 |
| 34.74 | 303 | 216 | 416 | 246 |
| 34.76 | 331 | 246 | 420 | 196 |
| 34.78 | 310 | 225 | 404 | 250 |
| 34.8 | 365 | 219 | 428 | 276 |
| 34.82 | 310 | 246 | 471 | 262 |
| 34.84 | 357 | 199 | 428 | 259 |
| 34.86 | 331 | 259 | 458 | 272 |
| 34.88 | 346 | 225 | 506 | 296 |
| 34.9 | 313 | 225 | 441 | 250 |
| 34.92 | 259 | 266 | 480 | 240 |
| 34.94 | 243 | 231 | 480 | 272 |
| 34.96 | 234 | 259 | 506 | 279 |
| 34.98 | 240 | 262 | 484 | 299 |
| 35 | 202 | 216 | 471 | 269 |
| 35.02 | 185 | 266 | 462 | 282 |
| 35.04 | 177 | 231 | 502 | 266 |
| 35.06 | 180 | 231 | 497 | 246 |
| 35.08 | 193 | 243 | 437 | 289 |
| 35.1 | 182 | 234 | 484 | 306 |
| 35.12 | 204 | 246 | 441 | 286 |
| 35.14 | 204 | 250 | 428 | 346 |
| 35.16 | 193 | 299 | 400 | 376 |
| 35.18 | 219 | 292 | 404 | 372 |
| 35.2 | 213 | 282 | 408 | 392 |
| 35.22 | 246 | 320 | 420 | 420 |
| 35.24 | 256 | 310 | 396 | 475 |
| 35.26 | 276 | 365 | 339 | 493 |
| 35.28 | 250 | 361 | 384 | 471 |
| 35.3 | 259 | 384 | 380 | 529 |
| 35.32 | 282 | 400 | 335 | 511 |
| 35.34 | 292 | 408 | 353 | 576 |
| 35.36 | 272 | 428 | 357 | 562 |
| 35.38 | 269 | 454 | 350 | 660 |
| 35.4 | 292 | 467 | 376 | 666 |
| 35.42 | 320 | 462 | 328 | 734 |
| 35.44 | 320 | 416 | 380 | 745 |
| 35.46 | 320 | 416 | 369 | 784 |
| 35.48 | 328 | 412 | 396 | 853 |
| 35.5 | 320 | 408 | 369 | 824 |
| 35.52 | 317 | 424 | 365 | 847 |
| 35.54 | 324 | 502 | 376 | 900 |
| 35.56 | 282 | 576 | 369 | 949 |
| 35.58 | 292 | 586 | 353 | 1030 |
| 35.6 | 286 | 529 | 365 | 1069 |
| 35.62 | 262 | 396 | 376 | 992 |
| 35.64 | 266 | 353 | 357 | 924 |
| 35.66 | 279 | 306 | 404 | 767 |
| 35.68 | 250 | 292 | 416 | 581 |
| 35.7 | 225 | 266 | 388 | 502 |
| 35.72 | 213 | 199 | 412 | 441 |
| 35.74 | 256 | 210 | 324 | 365 |
| 35.76 | 250 | 154 | 380 | 317 |
| 35.78 | 282 | 149 | 388 | 266 |
| 35.8 | 282 | 164 | 346 | 250 |
| 35.82 | 256 | 159 | 339 | 237 |
| 35.84 | 286 | 144 | 335 | 458 |
| 35.86 | 292 | 177 | 357 | 256 |
| 35.88 | 303 | 130 | 342 | 222 |
| 35.9 | 286 | 149 | 342 | 240 |
| 35.92 | 262 | 132 | 324 | 246 |
| 35.94 | 234 | 137 | 339 | 210 |
| 35.96 | 225 | 128 | 328 | 216 |
| 35.98 | 190 | 130 | 342 | 190 |
| 36 | 166 | 112 | 299 | 164 |
| 36.02 | 174 | 130 | 313 | 146 |
| 36.04 | 146 | 106 | 342 | 166 |
| 36.06 | 137 | 110 | 320 | 137 |
| 36.08 | 130 | 85 | 324 | 142 |
| 36.1 | 132 | 94 | 328 | 151 |
| 36.12 | 135 | 110 | 342 | 112 |
| 36.14 | 112 | 114 | 324 | 130 |
| 36.16 | 149 | 144 | 289 | 125 |
| 36.18 | 130 | 102 | 328 | 112 |
| 36.2 | 121 | 108 | 310 | 125 |
| 36.22 | 142 | 110 | 320 | 128 |
| 36.24 | 151 | 117 | 289 | 139 |
| 36.26 | 146 | 88 | 335 | 128 |
| 36.28 | 135 | 121 | 324 | 142 |
| 36.3 | 137 | 104 | 361 | 137 |
| 36.32 | 146 | 253 | 324 | 125 |
| 36.34 | 149 | 146 | 346 | 125 |
| 36.36 | 135 | 119 | 317 | 142 |
| 36.38 | 161 | 128 | 331 | 146 |
| 36.4 | 146 | 149 | 353 | 151 |
| 36.42 | 169 | 144 | 365 | 159 |
| 36.44 | 161 | 132 | 365 | 169 |
| 36.46 | 154 | 159 | 380 | 159 |
| 36.48 | 144 | 161 | 357 | 182 |
| 36.5 | 159 | 146 | 353 | 188 |
| 36.52 | 169 | 182 | 424 | 177 |
| 36.54 | 177 | 177 | 396 | 164 |
| 36.56 | 182 | 177 | 376 | 188 |
| 36.58 | 159 | 196 | 380 | 193 |
| 36.6 | 159 | 199 | 420 | 303 |
| 36.62 | 154 | 219 | 412 | 199 |
| 36.64 | 151 | 202 | 420 | 216 |
| 36.66 | 182 | 193 | 404 | 216 |
| 36.68 | 169 | 193 | 437 | 210 |
| 36.7 | 169 | 210 | 424 | 207 |
| 36.72 | 161 | 185 | 449 | 213 |
| 36.74 | 166 | 213 | 416 | 243 |
| 36.76 | 166 | 177 | 433 | 219 |
| 36.78 | 177 | 185 | 412 | 225 |
| 36.8 | 185 | 190 | 404 | 219 |
| 36.82 | 182 | 154 | 408 | 202 |
| 36.84 | 216 | 132 | 449 | 207 |
| 36.86 | 174 | 164 | 400 | 213 |
| 36.88 | 222 | 144 | 428 | 190 |
| 36.9 | 207 | 130 | 437 | 161 |
| 36.92 | 250 | 164 | 388 | 177 |
| 36.94 | 225 | 137 | 420 | 149 |
| 36.96 | 204 | 149 | 412 | 199 |
| 36.98 | 207 | 139 | 428 | 180 |
| 37 | 225 | 164 | 408 | 182 |
| 37.02 | 228 | 151 | 416 | 169 |
| 37.04 | 231 | 142 | 404 | 177 |
| 37.06 | 219 | 156 | 388 | 207 |
| 37.08 | 219 | 139 | 433 | 196 |
| 37.1 | 234 | 123 | 412 | 199 |
| 3T.12 | 207 | 130 | 353 | 182 |
| 37.14 | 234 | 128 | 388 | 177 |
| 37.16 | 216 | 121 | 396 | 144 |
| 37.18 | 216 | 125 | 400 | 161 |
| 37.2 | 193 | 121 | 416 | 154 |
| 37.22 | 207 | 125 | 404 | 159 |
| 37.24 | 234 | 149 | 392 | 164 |
| 37.26 | 225 | 149 | 376 | 174 |
| 37.28 | 222 | 142 | 424 | 182 |
| 37.3 | 216 | 117 | 384 | 177 |
| 37.32 | 234 | 156 | 369 | 166 |
| 37.34 | 228 | 159 | 365 | 216 |
| 37.36 | 237 | 174 | 372 | 196 |
| 37.38 | 243 | 164 | 350 | 182 |
| 37.4 | 276 | 177 | 392 | 199 |
| 37.42 | 262 | 156 | 369 | 196 |
| 37.44 | 279 | 210 | 420 | 234 |
| 37.46 | 266 | 202 | 384 | 243 |
| 37.48 | 225 | 202 | 357 | 250 |
| 37.5 | 243 | 207 | 361 | 259 |
| 37.52 | 222 | 228 | 361 | 292 |
| 37.54 | 202 | 225 | 392 | 289 |
| 37.56 | 177 | 269 | 408 | 331 |
| 37.58 | 207 | 243 | 346 | 317 |
| 37.6 | 169 | 262 | 376 | 331 |
| 37.62 | 177 | 234 | 357 | 339 |
| 37.64 | 137 | 225 | 372 | 299 |
| 37.66 | 139 | 234 | 357 | 350 |
| 37.68 | 161 | 216 | 408 | 335 |
| 37.7 | 142 | 234 | 365 | 240 |
| 37.72 | 146 | 237 | 372 | 250 |
| 37.74 | 156 | 225 | 384 | 216 |
| 37.76 | 146 | 204 | 357 | 240 |
| 37.78 | 190 | 188 | 412 | 246 |
| 37.8 | 159 | 182 | 396 | 231 |
| 37.82 | 202 | 196 | 408 | 219 |
| 37.84 | 193 | 207 | 412 | 234 |
| 37.86 | 172 | 199 | 392 | 250 |
| 37.88 | 172 | 199 | 408 | 250 |
| 37.9 | 177 | 196 | 384 | 231 |
| 37.92 | 193 | 177 | 392 | 222 |
| 37.94 | 169 | 169 | 380 | 246 |
| 37.96 | 177 | 196 | 400 | 222 |
| 37.98 | 185 | 202 | 408 | 243 |
| 38 | 193 | 180 | 376 | 207 |
| 38.02 | 177 | 174 | 388 | 199 |
| 38.04 | 177 | 172 | 384 | 210 |
| 38.06 | 156 | 161 | 380 | 177 |
| 38.08 | 219 | 161 | 404 | 159 |
| 38.1 | 169 | 159 | 380 | 172 |
| 38.12 | 166 | 169 | 392 | 166 |
| 38.14 | 199 | 177 | 396 | 199 |
| 38.16 | 202 | 154 | 392 | 185 |
| 38.18 | 225 | 172 | 404 | 169 |
| 38.2 | 282 | 196 | 412 | 182 |
| 38.22 | 213 | 169 | 388 | 196 |
| 38.24 | 180 | 169 | 365 | 185 |
| 38.26 | 269 | 174 | 350 | 196 |
| 38.28 | 199 | 199 | 384 | 174 |
| 38.3 | 193 | 182 | 412 | 185 |
| 38.32 | 202 | 185 | 384 | 199 |
| 38.34 | 199 | 185 | 380 | 202 |
| 38.36 | 202 | 156 | 369 | 219 |
| 38.38 | 237 | 151 | 324 | 174 |
| 38.4 | 292 | 166 | 369 | 169 |
| 38.42 | 266 | 199 | 388 | 196 |
| 38.44 | 253 | 199 | 380 | 182 |
| 38.46 | 262 | 166 | 380 | 196 |
| 38.48 | 246 | 182 | 424 | 188 |
| 38.5 | 259 | 199 | 392 | 180 |
| 38.52 | 222 | 202 | 404 | 213 |
| 38.54 | 231 | 182 | 392 | 219 |
| 38.56 | 225 | 188 | 372 | 185 |
| 38.58 | 234 | 210 | 369 | 199 |
| 38.6 | 246 | 219 | 412 | 196 |
| 38.62 | 259 | 193 | 396 | 199 |
| 38.64 | 262 | 207 | 400 | 228 |
| 38.66 | 231 | 231 | 404 | 250 |
| 38.68 | 286 | 210 | 400 | 259 |
| 38.7 | 219 | 199 | 372 | 222 |
| 38.72 | 216 | 193 | 384 | 225 |
| 38.74 | 193 | 142 | 396 | 219 |
| 38.76 | 196 | 196 | 328 | 207 |
| 38.78 | 213 | 166 | 384 | 188 |
| 38.8 | 199 | 164 | 384 | 202 |
| 38.82 | 193 | 159 | 361 | 196 |
| 38.84 | 193 | 139 | 369 | 190 |
| 38.86 | 174 | 132 | 369 | 177 |
| 38.88 | 169 | 132 | 380 | 174 |
| 38.9 | 169 | 156 | 372 | 188 |
| 38.92 | 151 | 130 | 346 | 149 |
| 38.94 | 154 | 144 | 388 | 190 |
| 38.96 | 142 | 139 | 380 | 210 |
| 38.98 | 159 | 159 | 361 | 188 |
| 39 | 159 | 146 | 365 | 216 |
| 39.02 | 159 | 159 | 357 | 207 |
| 39.04 | 182 | 149 | 320 | 216 |
| 39.06 | 164 | 135 | 346 | 219 |
| 39.08 | 146 | 151 | 320 | 210 |
| 39.1 | 142 | 151 | 339 | 234 |
| 39.12 | 169 | 149 | 331 | 188 |
| 39.14 | 156 | 137 | 331 | 190 |
| 39.16 | 174 | 151 | 353 | 177 |
| 39.18 | 144 | 135 | 335 | 177 |
| 39.2 | 161 | 110 | 335 | 154 |
| 39.22 | 174 | 144 | 303 | 154 |
| 39.24 | 149 | 139 | 335 | 185 |
| 39.26 | 154 | 119 | 317 | 139 |
| 39.28 | 164 | 108 | 328 | 125 |
| 39.3 | 146 | 144 | 299 | 139 |
| 39.32 | 177 | 100 | 339 | 130 |
| 39.34 | 169 | 128 | 342 | 161 |
| 39.36 | 182 | 121 | 320 | 121 |
| 39.38 | 188 | 128 | 299 | 128 |
| 39.4 | 199 | 121 | 317 | 130 |
| 39.42 | 172 | 125 | 296 | 112 |
| 39.44 | 174 | 132 | 331 | 154 |
| 39.46 | 204 | 144 | 313 | 132 |
| 39.48 | 188 | 119 | 380 | 149 |
| 39.5 | 182 | 149 | 365 | 128 |
| 39.52 | 169 | 108 | 350 | 156 |
| 39.54 | 182 | 125 | 369 | 139 |
| 39.56 | 207 | 137 | 365 | 142 |
| 39.58 | 174 | 137 | 369 | 144 |
| 39.6 | 185 | 154 | 342 | 142 |
| 39.62 | 199 | 144 | 388 | 164 |
| 39.64 | 204 | 130 | 384 | 144 |
| 39.66 | 207 | 149 | 372 | 149 |
| 39.68 | 219 | 149 | 428 | 128 |
| 39.7 | 188 | 144 | 396 | 149 |
| 39.72 | 182 | 128 | 384 | 130 |
| 39.74 | 196 | 125 | 408 | 180 |
| 39.76 | 174 | 142 | 396 | 135 |
| 39.78 | 180 | 125 | 388 | 154 |
| 39.8 | 164 | 156 | 433 | 177 |
| 39.82 | 182 | 128 | 384 | 137 |
| 39.84 | 161 | 125 | 369 | 142 |
| 39.86 | 174 | 132 | 416 | 132 |
| 39.88 | 151 | 125 | 388 | 142 |
| 39.9 | 146 | 114 | 408 | 130 |
| 39.92 | 142 | 151 | 408 | 112 |
| 39.94 | 164 | 137 | 400 | 151 |
| 39.96 | 182 | 114 | 416 | 144 |
| 39.98 | 199 | 125 | 388 | 144 |
| 40 | 196 | 125 | 416 | 130 |
| 40.02 | 193 | 135 | 396 | 135 |
| 40.04 | 213 | 135 | 380 | 144 |
| 40.06 | 234 | 151 | 404 | 144 |
| 40.08 | 231 | 159 | 424 | 142 |
| 40.1 | 240 | 149 | 412 | 137 |
| 40.12 | 256 | 144 | 400 | 151 |
| 40.14 | 231 | 130 | 384 | 156 |
| 40.16 | 279 | 146 | 424 | 142 |
| 40.18 | 317 | 139 | 372 | 161 |
| 40.2 | 335 | 156 | 404 | 135 |
| 40.22 | 342 | 164 | 396 | 149 |
| 40.24 | 335 | 172 | 388 | 149 |
| 40.26 | 342 | 159 | 396 | 169 |
| 40.28 | 357 | 180 | 376 | 164 |
| 40.3 | 433 | 149 | 392 | 159 |
| 40.32 | 424 | 156 | 388 | 156 |
| 40.34 | 437 | 177 | 384 | 151 |
| 40.36 | 412 | 149 | 396 | 159 |
| 40.38 | 396 | 172 | 400 | 156 |
| 40.4 | 424 | 164 | 428 | 144 |
| 40.42 | 454 | 151 | 404 | 128 |
| 40.44 | 428 | 149 | 408 | 144 |
| 40.46 | 388 | 154 | 392 | 161 |
| 40.48 | 282 | 185 | 420 | 262 |
| 40.5 | 303 | 139 | 380 | 151 |
| 40.52 | 292 | 154 | 392 | 149 |
| 40.54 | 286 | 180 | 392 | 174 |
| 40.56 | 269 | 149 | 380 | 156 |
| 40.58 | 237 | 161 | 380 | 185 |
| 40.6 | 225 | 174 | 384 | 164 |
| 40.62 | 234 | 154 | 416 | 177 |
| 40.64 | 219 | 164 | 400 | 185 |
| 40.66 | 237 | 199 | 388 | 164 |
| 40.68 | 213 | 193 | 396 | 159 |
| 40.7 | 199 | 196 | 416 | 190 |
| 40.72 | 222 | 219 | 412 | 169 |
| 40.74 | 234 | 196 | 369 | 177 |
| 40.76 | 222 | 207 | 404 | 182 |
| 40.78 | 361 | 228 | 384 | 169 |
| 40.8 | 193 | 240 | 396 | 188 |
| 40.82 | 196 | 213 | 384 | 180 |
| 40.84 | 240 | 231 | 384 | 199 |
| 40.86 | 231 | 243 | 412 | 207 |
| 40.88 | 266 | 237 | 388 | 213 |
| 40.9 | 234 | 292 | 396 | 222 |
| 40.92 | 253 | 259 | 392 | 231 |
| 40.94 | 282 | 289 | 396 | 225 |
| 40.96 | 303 | 292 | 404 | 250 |
| 40.98 | 303 | 296 | 412 | 250 |
| 41 | 317 | 299 | 400 | 237 |
| 41.02 | 286 | 328 | 449 | 246 |
| 41.04 | 262 | 306 | 404 | 240 |
| 41.06 | 289 | 262 | 408 | 213 |
| 41.08 | 328 | 266 | 396 | 231 |
| 41.1 | 339 | 253 | 384 | 231 |
| 41.12 | 310 | 246 | 408 | 231 |
| 41.14 | 286 | 243 | 400 | 225 |
| 41.16 | 292 | 240 | 392 | 222 |
| 41.18 | 250 | 182 | 400 | 199 |
| 41.2 | 246 | 296 | 416 | 174 |
| 41.22 | 228 | 188 | 400 | 182 |
| 41.24 | 210 | 185 | 388 | 174 |
| 41.26 | 207 | 193 | 400 | 193 |
| 41.28 | 193 | 180 | 416 | 164 |
| 41.3 | 193 | 188 | 380 | 193 |
| 41.32 | 169 | 193 | 404 | 180 |
| 41.34 | 174 | 185 | 428 | 210 |
| 41.36 | 180 | 182 | 433 | 182 |
| 41.38 | 199 | 177 | 458 | 225 |
| 41.4 | 177 | 185 | 454 | 193 |
| 41.42 | 174 | 185 | 467 | 164 |
| 41.44 | 185 | 172 | 441 | 169 |
| 41.46 | 193 | 198 | 471 | 213 |
| 41.48 | 199 | 193 | 445 | 193 |
| 41.5 | 213 | 204 | 467 | 182 |
| 41.52 | 207 | 190 | 484 | 210 |
| 41.54 | 196 | 202 | 462 | 185 |
| 41.56 | 202 | 182 | 488 | 228 |
| 41.58 | 216 | 166 | 475 | 182 |
| 41.6 | 193 | 207 | 449 | 210 |
| 41.62 | 190 | 188 | 467 | 182 |
| 41.64 | 172 | 151 | 454 | 196 |
| 41.66 | 225 | 177 | 454 | 199 |
| 41.68 | 213 | 188 | 467 | 207 |
| 41.7 | 204 | 169 | 420 | 228 |
| 41.72 | 202 | 193 | 475 | 237 |
| 41.74 | 225 | 207 | 428 | 259 |
| 41.76 | 216 | 210 | 416 | 324 |
| 41.78 | 204 | 199 | 437 | 250 |
| 41.8 | 210 | 207 | 420 | 213 |
| 41.82 | 228 | 237 | 392 | 269 |
| 41.84 | 228 | 237 | 376 | 253 |
| 41.86 | 243 | 243 | 380 | 286 |
| 41.88 | 237 | 219 | 357 | 272 |
| 41.9 | 234 | 216 | 372 | 259 |
| 41.92 | 225 | 196 | 392 | 259 |
| 41.94 | 231 | 246 | 353 | 250 |
| 41.96 | 250 | 250 | 353 | 259 |
| 41.98 | 272 | 256 | 346 | 259 |
| 42 | 262 | 231 | 335 | 286 |
| 42.02 | 250 | 276 | 365 | 282 |
| 42.04 | 225 | 259 | 353 | 272 |
| 42.06 | 219 | 276 | 384 | 313 |
| 42.08 | 240 | 228 | 320 | 262 |
| 42.1 | 228 | 259 | 416 | 246 |
| 42.12 | 216 | 216 | 380 | 243 |
| 42.14 | 219 | 207 | 353 | 256 |
| 42.16 | 188 | 188 | 384 | 231 |
| 42.18 | 193 | 174 | 408 | 213 |
| 42.2 | 193 | 180 | 384 | 202 |
| 42.22 | 172 | 166 | 380 | 193 |
| 42.24 | 169 | 144 | 437 | 172 |
| 42.26 | 182 | 154 | 416 | 180 |
| 42.28 | 188 | 156 | 416 | 199 |
| 42.3 | 159 | 132 | 420 | 172 |
| 42.32 | 144 | 159 | 428 | 139 |
| 42.34 | 146 | 135 | 396 | 166 |
| 42.36 | 139 | 112 | 424 | 139 |
| 42.38 | 146 | 117 | 441 | 154 |
| 42.4 | 135 | 137 | 437 | 139 |
| 42.42 | 159 | 144 | 420 | 182 |
| 42.44 | 125 | 128 | 437 | 159 |
| 42.46 | 139 | 130 | 416 | 164 |
| 42.48 | 128 | 130 | 467 | 154 |
| 42.5 | 139 | 154 | 475 | 154 |
| 42.52 | 154 | 135 | 433 | 166 |
| 42.54 | 132 | 146 | 408 | 151 |
| 42.56 | 146 | 159 | 441 | 144 |
| 42.58 | 159 | 142 | 454 | 193 |
| 42.6 | 139 | 149 | 424 | 177 |
| 42.62 | 137 | 166 | 396 | 146 |
| 42.64 | 137 | 174 | 424 | 182 |
| 42.66 | 151 | 174 | 441 | 177 |
| 42.68 | 159 | 169 | 428 | 188 |
| 42.7 | 159 | 185 | 454 | 219 |
| 42.72 | 166 | 177 | 449 | 207 |
| 42.74 | 174 | 164 | 404 | 207 |
| 42.76 | 172 | 185 | 408 | 207 |
| 42.78 | 177 | 185 | 433 | 262 |
| 42.8 | 177 | 196 | 424 | 266 |
| 42.82 | 169 | 199 | 454 | 276 |
| 42.84 | 177 | 262 | 467 | 259 |
| 42.86 | 161 | 234 | 396 | 299 |
| 42.88 | 164 | 210 | 420 | 272 |
| 42.9 | 156 | 222 | 408 | 272 |
| 42.92 | 159 | 234 | 392 | 331 |
| 42.94 | 144 | 246 | 449 | 299 |
| 42.96 | 174 | 231 | 416 | 342 |
| 42.98 | 164 | 250 | 404 | 306 |
| 43 | 182 | 237 | 404 | 320 |
| 43.02 | 196 | 269 | 433 | 328 |
| 43.04 | 159 | 272 | 372 | 376 |
| 43.06 | 180 | 276 | 437 | 353 |
| 43.08 | 174 | 299 | 396 | 400 |
| 43.1 | 193 | 303 | 388 | 445 |
| 43.12 | 185 | 331 | 380 | 467 |
| 43.14 | 177 | 324 | 502 | 454 |
| 43.16 | 199 | 310 | 388 | 484 |
| 43.18 | 188 | 353 | 398 | 538 |
| 43.2 | 210 | 346 | 392 | 534 |
| 43.22 | 182 | 388 | 365 | 552 |
| 43.24 | 222 | 346 | 339 | 534 |
| 43.26 | 207 | 388 | 331 | 534 |
| 43.28 | 216 | 384 | 376 | 534 |
| 43.3 | 207 | 400 | 380 | 538 |
| 43.32 | 222 | 404 | 353 | 524 |
| 43.34 | 216 | 372 | 342 | 543 |
| 43.36 | 216 | 396 | 335 | 571 |
| 43.38 | 234 | 384 | 372 | 538 |
| 43.4 | 190 | 416 | 320 | 566 |
| 43.42 | 216 | 467 | 350 | 566 |
| 43.44 | 219 | 400 | 310 | 605 |
| 43.46 | 225 | 480 | 342 | 605 |
| 43.48 | 240 | 437 | 339 | 610 |
| 43.5 | 240 | 441 | 335 | 635 |
| 43.52 | 243 | 480 | 320 | 660 |
| 43.54 | 250 | 441 | 310 | 655 |
| 43.56 | 250 | 449 | 317 | 645 |
| 43.58 | 262 | 506 | 331 | 660 |
| 43.6 | 259 | 416 | 324 | 581 |
| 43.62 | 253 | 342 | 303 | 581 |
| 43.64 | 219 | 433 | 346 | 506 |
| 43.66 | 216 | 320 | 320 | 445 |
| 43.68 | 204 | 306 | 328 | 416 |
| 43.7 | 240 | 269 | 286 | 376 |
| 43.72 | 185 | 234 | 331 | 369 |
| 43.74 | 202 | 225 | 335 | 328 |
| 43.76 | 193 | 199 | 320 | 310 |
| 43.78 | 154 | 225 | 313 | 262 |
| 43.8 | 159 | 204 | 320 | 253 |
| 43.82 | 193 | 225 | 286 | 237 |
| 43.84 | 180 | 228 | 282 | 202 |
| 43.86 | 156 | 210 | 313 | 193 |
| 43.88 | 185 | 193 | 346 | 202 |
| 43.9 | 202 | 216 | 299 | 228 |
| 43.92 | 185 | 196 | 313 | 196 |
| 43.94 | 159 | 207 | 299 | 210 |
| 43.96 | 188 | 204 | 353 | 207 |
| 43.98 | 196 | 199 | 324 | 202 |
| 44 | 161 | 199 | 299 | 202 |
| 44.02 | 166 | 190 | 279 | 193 |
| 44.04 | 164 | 182 | 335 | 185 |
| 44.06 | 188 | 180 | 306 | 174 |
| 44.08 | 213 | 177 | 339 | 169 |
| 44.1 | 196 | 159 | 328 | 151 |
| 44.12 | 210 | 154 | 328 | 177 |
| 44.14 | 180 | 169 | 357 | 154 |
| 44.16 | 204 | 159 | 342 | 146 |
| 44.18 | 196 | 161 | 342 | 169 |
| 44.2 | 196 | 154 | 357 | 169 |
| 44.22 | 196 | 146 | 571 | 174 |
| 44.24 | 149 | 128 | 372 | 164 |
| 44.26 | 177 | 169 | 357 | 159 |
| 44.28 | 180 | 137 | 357 | 174 |
| 44.3 | 149 | 159 | 350 | 188 |
| 44.32 | 169 | 177 | 346 | 149 |
| 44.34 | 156 | 185 | 376 | 151 |
| 44.36 | 190 | 166 | 342 | 174 |
| 44.38 | 154 | 185 | 384 | 174 |
| 44.4 | 154 | 188 | 361 | 169 |
| 44.42 | 159 | 199 | 380 | 240 |
| 44.44 | 137 | 202 | 342 | 185 |
| 44.46 | 137 | 213 | 313 | 199 |
| 44.48 | 159 | 199 | 365 | 222 |
| 44.5 | 156 | 237 | 335 | 213 |
| 44.52 | 139 | 231 | 365 | 243 |
| 44.54 | 135 | 225 | 353 | 216 |
| 44.56 | 154 | 253 | 361 | 282 |
| 44.58 | 146 | 262 | 324 | 246 |
| 44.6 | 159 | 234 | 361 | 266 |
| 44.62 | 151 | 246 | 369 | 292 |
| 44.64 | 159 | 286 | 376 | 299 |
| 44.66 | 177 | 253 | 350 | 310 |
| 44.68 | 156 | 276 | 369 | 296 |
| 44.7 | 174 | 259 | 342 | 303 |
| 44.72 | 156 | 272 | 331 | 303 |
| 44.74 | 142 | 272 | 353 | 269 |
| 44.76 | 180 | 240 | 324 | 328 |
| 44.78 | 177 | 266 | 339 | 286 |
| 44.8 | 16fi | 272 | 342 | 286 |
| 44.82 | 149 | 262 | 328 | 282 |
| 44.84 | 151 | 237 | 339 | 286 |
| 44.86 | 132 | 246 | 342 | 292 |
| 44.88 | 146 | 276 | 369 | 303 |
| 44.9 | 149 | 228 | 384 | 299 |
| 44.92 | 125 | 262 | 350 | 310 |
| 44.94 | 149 | 234 | 350 | 279 |
| 44.96 | 144 | 266 | 353 | 303 |
| 44.98 | 144 | 193 | 350 | 262 |
| 45 | 156 | 196 | 317 | 286 |
| 45.02 | 146 | 199 | 350 | 231 |
| 45.04 | 156 | 185 | 331 | 213 |
| 45.06 | 159 | 161 | 306 | 210 |
| 45.08 | 149 | 174 | 299 | 202 |
| 45.1 | 151 | 156 | 310 | 182 |
| 45.12 | 144 | 144 | 296 | 180 |
| 45.14 | 161 | 151 | 317 | 164 |
| 45.16 | 169 | 166 | 320 | 146 |
| 45.18 | 166 | 180 | 289 | 169 |
| 45.2 | 177 | 180 | 286 | 172 |
| 45.22 | 169 | 146 | 328 | 174 |
| 45.24 | 185 | 159 | 269 | 169 |
| 45.26 | 188 | 139 | 306 | 149 |
| 45.28 | 169 | 159 | 310 | 177 |
| 45.3 | 190 | 169 | 320 | 185 |
| 45.32 | 190 | 151 | 292 | 161 |
| 45.34 | 188 | 166 | 289 | 182 |
| 45.36 | 182 | 146 | 286 | 202 |
| 45.38 | 193 | 172 | 306 | 193 |
| 45.4 | 196 | 193 | 289 | 172 |
| 45.42 | 182 | 161 | 276 | 193 |
| 45.44 | 172 | 177 | 317 | 188 |
| 45.46 | 169 | 188 | 272 | 196 |
| 45.48 | 185 | 149 | 262 | 219 |
| 45.5 | 156 | 135 | 296 | 199 |
| 45.52 | 159 | 169 | 279 | 174 |
| 45.54 | 169 | 154 | 317 | 182 |
| 45.56 | 177 | 154 | 272 | 166 |
| 45.58 | 164 | 154 | 289 | 164 |
| 45.6 | 174 | 149 | 276 | 149 |
| 45.62 | 161 | 156 | 289 | 193 |
| 45.64 | 172 | 164 | 266 | 180 |
| 45.66 | 188 | 154 | 266 | 213 |
| 45.68 | 151 | 172 | 299 | 188 |
| 45.7 | 149 | 213 | 276 | 164 |
| 45.72 | 207 | 169 | 299 | 193 |
| 45.74 | 237 | 180 | 279 | 213 |
| 45.76 | 199 | 188 | 296 | 196 |
| 45.78 | 196 | 190 | 272 | 196 |
| 45.8 | 185 | 219 | 282 | 193 |
| 45.82 | 204 | 204 | 296 | 202 |
| 45.84 | 172 | 219 | 279 | 207 |
| 45.86 | 185 | 210 | 272 | 204 |
| 45.88 | 216 | 193 | 276 | 219 |
| 45.9 | 196 | 188 | 286 | 237 |
| 45.92 | 196 | 188 | 276 | 219 |
| 45.94 | 225 | 225 | 292 | 213 |
| 45.96 | 240 | 204 | 286 | 222 |
| 45.98 | 174 | 193 | 296 | 234 |
| 46 | 169 | 199 | 292 | 213 |
| 46.02 | 166 | 166 | 266 | 216 |
| 46.04 | 193 | 164 | 269 | 243 |
| 46.06 | 185 | 331 | 269 | 240 |
| 46.08 | 161 | 146 | 292 | 219 |
| 46.1 | 164 | 159 | 259 | 190 |
| 46.12 | 159 | 154 | 243 | 231 |
| 46.14 | 139 | 135 | 262 | 216 |
| 46.16 | 289 | 128 | 250 | 161 |
| 46.18 | 185 | 128 | 262 | 164 |
| 46.2 | 159 | 135 | 286 | 185 |
| 46.22 | 151 | 137 | 292 | 161 |
| 46.24 | 169 | 130 | 299 | 156 |
| 46.26 | 154 | 114 | 286 | 139 |
| 46.28 | 137 | 117 | 299 | 125 |
| 46.3 | 137 | 117 | 286 | 137 |
| 46.32 | 135 | 137 | 296 | 125 |
| 46.34 | 132 | 144 | 282 | 130 |
| 46.36 | 121 | 182 | 303 | 137 |
| 46.38 | 156 | 125 | 262 | 142 |
| 46.4 | 139 | 139 | 286 | 154 |
| 46.42 | 144 | 119 | 292 | 130 |
| 46.44 | 142 | 154 | 299 | 137 |
| 46.46 | 125 | 137 | 276 | 156 |
| 46.48 | 130 | 149 | 289 | 161 |
| 46.5 | 132 | 154 | 272 | 161 |
| 46.52 | 130 | 159 | 266 | 154 |
| 46.54 | 154 | 154 | 259 | 156 |
| 46.56 | 121 | 166 | 292 | 142 |
| 46.58 | 149 | 142 | 282 | 135 |
| 46.6 | 154 | 154 | 269 | 142 |
| 46.62 | 161 | 159 | 276 | 159 |
| 46.64 | 161 | 146 | 276 | 161 |
| 46.66 | 149 | 142 | 272 | 166 |
| 46.68 | 149 | 169 | 299 | 164 |
| 46.7 | 174 | 154 | 286 | 154 |
| 46.72 | 151 | 172 | 276 | 146 |
| 46.74 | 159 | 139 | 259 | 135 |
| 46.76 | 159 | 144 | 246 | 169 |
| 46.78 | 159 | 146 | 292 | 159 |
| 46.8 | 128 | 149 | 282 | 180 |
| 46.82 | 142 | 159 | 262 | 154 |
| 46.84 | 154 | 142 | 246 | 156 |
| 46.88 | 137 | 161 | 243 | 174 |
| 46.88 | 125 | 139 | 262 | 142 |
| 46.9 | 146 | 159 | 237 | 164 |
| 46.92 | 130 | 142 | 243 | 156 |
| 46.94 | 137 | 144 | 266 | 125 |
| 46.96 | 132 | 159 | 228 | 164 |
| 46.98 | 151 | 149 | 253 | 156 |
| 47 | 117 | 180 | 269 | 164 |
| 47.02 | 123 | 177 | 259 | 154 |
| 47.04 | 146 | 199 | 250 | 159 |
| 47.06 | 128 | 180 | 234 | 172 |
| 47.08 | 125 | 154 | 228 | 177 |
| 47.1 | 144 | 177 | 250 | 185 |
| 47.12 | 130 | 180 | 243 | 174 |
| 47.14 | 121 | 177 | 269 | 177 |
| 47.16 | 144 | 204 | 228 | 193 |
| 47.18 | 137 | 188 | 266 | 169 |
| 47.2 | 130 | 180 | 240 | 164 |
| 47.22 | 154 | 174 | 240 | 185 |
| 47.24 | 144 | 180 | 259 | 182 |
| 47.26 | 169 | 190 | 253 | 180 |
| 47.28 | 164 | 159 | 262 | 182 |
| 47.3 | 193 | 166 | 216 | 166 |
| 47.32 | 166 | 174 | 259 | 172 |
| 47.34 | 182 | 164 | 222 | 174 |
| 47.36 | 161 | 149 | 231 | 169 |
| 47.38 | 164 | 151 | 243 | 156 |
| 47.4 | 174 | 139 | 234 | 182 |
| 47.42 | 182 | 159 | 256 | 169 |
| 47.44 | 182 | 139 | 225 | 161 |
| 47.46 | 188 | 123 | 259 | 159 |
| 47.48 | 193 | 156 | 262 | 159 |
| 47.5 | 164 | 125 | 240 | 154 |
| 47.52 | 188 | 121 | 262 | 166 |
| 47.54 | 185 | 132 | 237 | 161 |
| 47.56 | 193 | 142 | 219 | 149 |
| 47.58 | 177 | 137 | 250 | 159 |
| 47.6 | 177 | 144 | 237 | 159 |
| 47.62 | 156 | 123 | 228 | 172 |
| 47.64 | 164 | 144 | 237 | 172 |
| 47.66 | 159 | 130 | 259 | 190 |
| 47.68 | 161 | 139 | 246 | 180 |
| 47.7 | 161 | 130 | 237 | 169 |
| 47.72 | 151 | 156 | 225 | 213 |
| 47.74 | 154 | 156 | 262 | 216 |
| 47.76 | 130 | 144 | 237 | 185 |
| 47.78 | 130 | 130 | 234 | 174 |
| 47.8 | 123 | 164 | 246 | 210 |
| 47.82 | 156 | 121 | 210 | 188 |
| 47.84 | 137 | 130 | 253 | 149 |
| 47.86 | 146 | 123 | 250 | 164 |
| 47.88 | 137 | 121 | 243 | 174 |
| 47.9 | 164 | 128 | 231 | 135 |
| 47.92 | 151 | 139 | 246 | 121 |
| 47.94 | 144 | 135 | 228 | 146 |
| 47.96 | 149 | 110 | 259 | 144 |
| 47.98 | 142 | 121 | 272 | 121 |
| 48 | 132 | 119 | 253 | 132 |
| 48.02 | 132 | 121 | 240 | 149 |
| 48.04 | 139 | 110 | 243 | 144 |
| 48.06 | 132 | 96 | 272 | 137 |
| 48.08 | 125 | 135 | 256 | 135 |
| 48.1 | 130 | 121 | 253 | 144 |
| 48.12 | 123 | 112 | 282 | 128 |
| 48.14 | 142 | 100 | 253 | 123 |
| 48.16 | 137 | 123 | 259 | 146 |
| 48.18 | 125 | 125 | 272 | 123 |
| 48.2 | 130 | 119 | 262 | 128 |
| 48.22 | 146 | 100 | 246 | 149 |
| 48.24 | 139 | 132 | 259 | 121 |
| 48.26 | 137 | 110 | 240 | 137 |
| 48.28 | 139 | 114 | 292 | 130 |
| 48.3 | 151 | 137 | 266 | 128 |
| 48.32 | 161 | 149 | 289 | 130 |
| 48.34 | 177 | 130 | 269 | 154 |
| 48.36 | 144 | 114 | 286 | 149 |
| 48.38 | 180 | 121 | 259 | 128 |
| 48.4 | 149 | 146 | 296 | 159 |
| 48.42 | 156 | 144 | 262 | 154 |
| 48.44 | 121 | 151 | 266 | 159 |
| 48.46 | 166 | 139 | 266 | 135 |
| 48.48 | 144 | 193 | 231 | 169 |
| 48.5 | 154 | 154 | 253 | 164 |
| 48.52 | 132 | 154 | 269 | 166 |
| 48.54 | 135 | 151 | 276 | 159 |
| 48.56 | 139 | 161 | 246 | 161 |
| 48.58 | 117 | 164 | 282 | 168 |
| 48.6 | 128 | 159 | 272 | 180 |
| 48.62 | 128 | 182 | 266 | 169 |
| 48.64 | 137 | 142 | 289 | 174 |
| 48.66 | 128 | 169 | 266 | 172 |
| 48.68 | 123 | 161 | 246 | 188 |
| 48.7 | 102 | 137 | 222 | 180 |
| 48.72 | 125 | 142 | 253 | 164 |
| 48.74 | 135 | 159 | 256 | 159 |
| 48.76 | 121 | 130 | 243 | 135 |
| 48.78 | 159 | 110 | 256 | 151 |
| 48.8 | 135 | 137 | 240 | 151 |
| 48.82 | 121 | 119 | 262 | 149 |
| 48.84 | 135 | 130 | 231 | 142 |
| 48.86 | 149 | 121 | 240 | 130 |
| 48.88 | 132 | 121 | 253 | 132 |
| 48.9 | 123 | 114 | 234 | 128 |
| 48.92 | 149 | 114 | 234 | 130 |
| 48.94 | 144 | 135 | 237 | 108 |
| 48.96 | 166 | 102 | 246 | 117 |
| 48.98 | 137 | 128 | 272 | 102 |
| 49 | 137 | 128 | 225 | 128 |
| 49.02 | 130 | 108 | 237 | 125 |
| 49.04 | 146 | 117 | 225 | 117 |
| 49.06 | 149 | 108 | 213 | 135 |
| 49.08 | 142 | 94 | 246 | 114 |
| 49.1 | 144 | 112 | 256 | 123 |
| 49.12 | 137 | 100 | 219 | 121 |
| 49.14 | 151 | 117 | 237 | 121 |
| 49.16 | 146 | 106 | 190 | 137 |
| 49.18 | 156 | 104 | 213 | 117 |
| 49.2 | 130 | 112 | 216 | 130 |
| 49.22 | 128 | 92 | 234 | 130 |
| 49.24 | 135 | 123 | 240 | 135 |
| 49.26 | 121 | 130 | 207 | 135 |
| 49.28 | 132 | 128 | 216 | 132 |
| 49.3 | 117 | 108 | 207 | 135 |
| 49.32 | 135 | 108 | 225 | 149 |
| 49.34 | 128 | 98 | 216 | 154 |
| 49.36 | 137 | 130 | 204 | 151 |
| 49.38 | 125 | 110 | 240 | 151 |
| 49.4 | 110 | 125 | 243 | 146 |
| 49.42 | 121 | 130 | 234 | 219 |
| 49.44 | 121 | 114 | 219 | 151 |
| 49.46 | 119 | 92 | 213 | 156 |
| 49.48 | 137 | 130 | 202 | 149 |
| 49.5 | 144 | 123 | 240 | 121 |
| 49.52 | 102 | 123 | 216 | 117 |
| 49.54 | 117 | 117 | 225 | 151 |
| 49.56 | 114 | 130 | 228 | 139 |
| 49.58 | 108 | 149 | 222 | 128 |
| 49.6 | 112 | 121 | 207 | 139 |
| 49.62 | 110 | 110 | 210 | 139 |
| 49.64 | 123 | 128 | 210 | 137 |
| 49.66 | 100 | 119 | 222 | 137 |
| 49.68 | 92 | 121 | 213 | 149 |
| 49.7 | 135 | 128 | 237 | 110 |
| 49.72 | 121 | 110 | 193 | 130 |
| 49.74 | 119 | 119 | 207 | 139 |
| 49.76 | 128 | 112 | 207 | 128 |
| 49.78 | 108 | 132 | 225 | 121 |
| 49.8 | 104 | 130 | 222 | 125 |
| 49.82 | 106 | 108 | 199 | 121 |
| 49.84 | 119 | 137 | 222 | 130 |
| 49.86 | 100 | 135 | 207 | 144 |
| 49.88 | 112 | 104 | 222 | 137 |
| 49.9 | 114 | 132 | 225 | 130 |
| 49.92 | 102 | 112 | 210 | 137 |
| 49.94 | 92 | 94 | 204 | 161 |
| 49.96 | 121 | 102 | 202 | 123 |
| 49.98 | 98 | 100 | 196 | 125 |
| 50 | | | | |

### TABLE B

2θ and d-spacing values of the significant XRPD peaks shown by arrows in Figure 15 for smilagenin in form VI (smilagenin channel hydrate). The relative intensity of each peak as a percentage of the intensity of the strongest peak is also shown. λ is 1.5406Å and the 2θ range is 2.4° to 27.5°. The measurements were conducted at 25°C (room temperature).

| Angle (2θ)/° | d/A | Relative intensity (%) |
|---|---|---|
| 3.463 | 25.49368 | 3.0 |
| 3.930 | 22.46393 | 7.1 |
| 6.467 | 13.65546 | 15.6 |
| 7.000 | 12.61750 | 3.6 |
| 7.851 | 11.25135 | 11.3 |
| 8.667 | 10.19453 | 3.2 |
| 10.130 | 8.72471 | 8.3 |
| 11.213 | 7.88477 | 3.5 |
| 11.844 | 7.46603 | 4.2 |
| 12.421 | 7.11998 | 8.7 |
| 13.007 | 6.80100 | 12.1 |
| 13.642 | 6.48582 | 20.4 |
| 13.959 | 6.33900 | 42.5 |
| 14.236 | 6.21630 | 29.5 |
| 14.625 | 6.05196 | 28.8 |
| 15.059 | 5.87850 | 23.5 |
| 15.566 | 5.68787 | 15.5 |
| 16.550 | 5.35198 | 24.2 |
| 16.900 | 5.24192 | 48.0 |
| 17.221 | 5.14492 | 100.0 |
| 17.818 | 4.97399 | 26.7 |
| 18.377 | 4.82384 | 34.3 |
| 19.354 | 4.58246 | 18.7 |
| 19.752 | 4.49103 | 19.8 |
| 20.312 | 4.36847 | 26.0 |
| 21.837 | 4.06663 | 16.2 |
| 23.125 | 3.84295 | 19.7 |
| 23.630 | 3.76205 | 15.8 |
| 24.904 | 3.57235 | 11.7 |
| 25.868 | 3.44136 | 17.0 |
| 26.540 | 3.35581 | 13.2 |
| 27.100 | 3.28765 | 12.4 |

### TABLE C

2θ and d-spacing values of the significant XRPD peaks shown by arrows in Figure 16 for smilagenin in form VII (smilagenin IPA solvate). The relative intensity of each peak as a percentage of the intensity of the strongest peak is also shown. λ is 1.5406Å and the 2θ range is 2.4° to 27.5°. The measurements were conducted at 25°C (room temperature).

| Angle (2θ)/° | *d*/Å | Relative intensity (%) |
|---|---|---|
| 6.459 | 13.67403 | 43.0 |
| 7.184 | 12.29531 | 18.4 |
| 10.215 | 8.65248 | 11.5 |
| 11.581 | 7.63461 | 25.4 |
| 12.639 | 6.99803 | 73.1 |
| 13.030 | 6.78879 | 100.0 |
| 13.658 | 6.47790 | 61.2 |
| 14.176 | 6.24260 | 93.2 |
| 15.150 | 5.84325 | 53.7 |
| 15.534 | 5.69963 | 82.5 |
| 16.378 | 5.40768 | 67.2 |
| 16.700 | 5.30424 | 56.8 |
| 17.276 | 5.12881 | 53.3 |
| 18.129 | 4.88935 | 51.4 |
| 18.820 | 4.71129 | 65.6 |
| 19.316 | 4.59146 | 60.0 |
| 20.334 | 4.38377 | 66.7 |
| 22.965 | 3.86941 | 44.9 |
| 23.523 | 3.77887 | 42.3 |
| 24.752 | 3.59400 | 37.5 |
| 25.448 | 3.49721 | 39.3 |

The foregoing broadly describes the present invention without limitation. Variations and modifications as will be readily apparent to those of ordinary skill in this art are intended to be covered by the present application and resultant patent(s).

## Claims

1. Smilagenin selected from the group consisting of smilagenin crystalline forms III, IIIA, V, VI and VII as defined herein.

2. Smilagenin channel hydrate.

3. Smilagenin monohydrate.

4. Smilagenin hydrate at a hydration stoichiometry other than 1:1.

5. Amorphous smilagenin.

6. A material according to any one of the preceeding claims, substantially free of another form of smilagenin and/or substantially free of other steroidal sapogenins and/or steroidal saponins.

7. A material according to any one of the preceeding claims in at least about 50
% by weight pure form.

8. A composition comprising a material according to any one of the preceeding claims in admixture with one or more further components selected from: one or more other materials according to any one of the preceeding claims, another form of smilagenin, any other biologically active material, and any biologically inactive material.

9. A material according to any of claims 1-7 for use as a medicament, foodstuff, food supplement or beverage.

10. A method of treatment of a human or non-human animal suffering from, or susceptible to, a condition selected from, high blood cholesterol levels, obesity and diabetes obesity syndromes, cognitive dysfunction and allied conditions, non-cognitive neurodegeneration, non-cognitive neuromuscular degeneration, motor-sensory neurodegeneration and loss of receptor function in the absence of cognitive, neural or neuromuscular impairment, which comprises administering to the said human or non-human animal and effective amount of a material or composition according to claims 1-8.

11. A method for obtaining pharmaceutical or edible grade smilagenin or a derivative thereof, wherein at least one step of the process includes preparing a material according to any one of claims 1-8.

12. A method of adjusting smilagenin between the amorphous form and the crystalline forms III, IIIA, V, VI and VII as defined herein, comprising precipitation of an adjusted form of smilagenin from a solution of a first such form of smilagenin in an appropriate organic solvent or solvent mixture, optionally in the presence of water, to obtain the adjusted form of smilagenin.

13. A method of adjusting the hydration level of smilagenin between the anhydrous, dihydrate and intermediate levels, comprising precipitation or other crystallisation of a first form of smilagenin from a solution thereof in an appropriate organic solvent or solvent mixture, optionally in the presence of water, to obtain smilagenin at a said adjusted hydration level.

14. A method of preparing a prodrug of smilagenin, which comprises esterifying a material according to any one of claims 1-7.

15. A prodrug of smilagenin, when obtained by a method according to claim 14.
